# EUROPEAN PATENT SPECIFICATION

(11) **EP 4 415 600 B1**
(45) Date of publication and mention of the grant of the patent: **19.03.2025**
(21) Application number: 23701542.5
(22) Date of filing: 25.01.2023
(51) Int. Cl.: A61B 3/02, A61B 3/024, A61B 3/028, A61B 3/06, A61B 3/032, A61B 3/00, A61B 3/113

(54) **METHOD AND APPARATUS FOR DETERMINING AT LEAST ONE VISUAL PARAMETER**
VERFAHREN UND VORRICHTUNG ZUR BESTIMMUNG VON MINDESTENS EINEM VISUELLEN PARAMETER
PROCÉDÉ ET APPAREIL PERMETTANT DE DÉTERMINER AU MOINS UN PARAMÈTRE VISUEL

(30) Priority: 26.01.2022 EP 22153366
(43) Date of publication of application: 21.08.2024
(73) Proprietor: Carl Zeiss Vision International GmbH, 73430 Aalen (DE)
(72) Inventor: RIFAI, Katharina, 72072 Tübingen (DE); WAHL, Siegfried, 73072 Donzdorf (DE); LEUBE, Alexander, 73431 Aalen (DE)
(74) Representative: Altmann Stößel Dick Patentanwälte PartG mbB
(86) International application number: PCT/EP2023/051771
(87) International publication number: WO 2023/144191

(56) References cited:
- EP-A1- 3 730 037
- WO-A1-2018/006013
- WO-A1-2022/015227
- US-A1- 2012 019 779
- US-A1- 2012 105 609
- US-A1- 2020 214 559
- US-B1- 10 945 599

## Description

### Field of the invention

The present invention relates to a computer-implemented method, a computer program and an apparatus for determining at least one visual parameter of at least one eye of a person.

### Related art

Bonnen, K., Burge, J., Yates, J., Pillow, J. and Cormack, L. K., Continuous psychophysics: Target-tracking to measure visual sensitivity, Journal of Vision (2015), 15(3):14, 1-16 describes the introduction of a novel framework for estimating visual sensitivity using a continuous target-tracking task in concert with a dynamic internal model of human visual performance. Observers used a mouse cursor to track the center of a two-dimensional Gaussian luminance blob as it moved in a random walk in a field of dynamic additive Gaussian luminance noise. To estimate visual sensitivity, they fit a Kalman filter model to the human tracking data under the assumption that humans behave as Bayesian ideal observers. Such observers optimally combine prior information with noisy observations to produce an estimate of target position at each time step. They found that estimates of human sensory noise obtained from the Kalman filter fit were highly correlated with traditional psychophysical measures of human sensitivity.

Collewijn, H. and Tamminga, E. P., Human smooth and saccadic eye movements during voluntary pursuit of different target motions on different backgrounds, Journal of Physiology (1984), 351, 217-250 describes that horizontal and vertical eye movements of human subjects were recorded with a scleral induction-coil technique during voluntary pursuit of sinusoidal, triangular and pseudo-random target motions of different frequency, amplitude and dimensionality upon a dark, diffuse or structured background. Data processing included separation of the composite eye movement into a cumulative smooth and saccadic displacement, computation of gain and phase of the composite and smooth eye movements with respect to the target movement and analysis of retinal position error.

Dakin, S. C. and Turnbull, P. R. K., Similar contrast sensitivity functions measured using psychophysics and optokinetic nystagmus, Scientific Reports (2016), 6:34514, describes that although the contrast sensitivity function (CSF) is a particularly useful way of characterising functional vision, its measurement relies on observers making reliable perceptual reports. Such procedures can be challenging when testing children. In the reference they further describe a system for measuring the CSF using an automated analysis of optokinetic nystagmus (OKN); an involuntary oscillatory eye movement made in response to drifting stimuli, here spatial-frequency (SF) band-pass noise.

Doustkouhi, S. M., Turnbull, P. R. K. and Dakin, S. C., The effect of refractive error on optokinetic nystagmus, Scientific Reports (2020), 10:20062 describes that subjective refraction is the gold-standard for prescribing refractive correction, but its accuracy is limited by patient's subjective judgment about their clarity of vision. They asked if an involuntary eye movement, optokinetic nystagmus (OKN), could serve as an objective measure of visual-clarity, specifically measuring the dependence of OKN - elicited by drifting spatial-frequency filtered noise - on mean spherical equivalent (MSE) refractive error. In a frist Experiment they quantified OKN score - a measure of consistency with stimulus-direction - for participants with different MSEs. In a second Experiment 2 they quantified the relationship between OKN gain and MSEs induced with contact lenses for each participant.

Harrison, J. J., Freeman, T. C. A. and Sumner, P., Saccade-like behavior in the fast-phases of optokinetic nystagmus: An illustration of the emergence of volitional actions from automatic reflexes, Journal of Experimental Psychology: General (2014), 143(5), 1923-1938 describes that a potential exemplar for understanding how volitional actions emerged from reflexes, they studied the relationship between an ancient reflexive gaze stabilization mechanism (optokinetic nystagmus [OKN]) and purposeful eye movements (saccades) that target an object. Traditionally, these have been considered distinct (except in the kinematics of their execution) and have been studied independently.

Lindner, A. and Ilg, U. J., Suppression of optokinesis during smooth pursuit eye movements revisited: The role of extra-retinal information. Vision Research (2006), 46(6-7), 761-767, describe that when eyes track objects that are moving in a richly structured environment, the retinal image of the stationary visual scene inevitably moves over the retina in a direction opposite to the eye movement. Such self-motion-induced global retinal slip usually provides an ideal stimulus for the optokinetic reflex. This reflex operates to compensate for global image flow. However, during smooth pursuit eye movements it must be shut down so that the reflex does not counteract the voluntary pursuit of moving targets.

Lindner, A., Schwarz, U. and Ilg, U. J., Cancellation of self-induced retinal image motion during smooth pursuit eye movements. Vision Research (2001), 41(13), 1685-1694 describes that when eyes are tracking a target that is moving in front of a structured background, global motion of equal speed is induced in the opposite direction. This effect has been termed reafference, which, astonishingly, does not significantly affect the execution of such pursuit eye movements. Employing brief and unexpected injections of full-field motion during ongoing human smooth pursuit, they demonstrate that the sensitivity for full-field motion is reduced strongly in the direction opposite to the eye movement, i.e. the direction of reafferent background motion. Their experiments further characterize this asymmetry in visual motion processing and provide a preliminary explanation for the accuracy of the pursuit system despite self-induced motion.

Masson, G., Proteau, L. and Mestre, D. R., Effects of stationary and moving textured backgrounds on the visuo-oculo-manual tracking in humans, Vision Research (1995), 35(6), 837-852 describes that they investigated the effects of stationary and moving textured backgrounds on ocular and manual pursuit of a discrete target that suddenly starts to move at constant speed (ramp motion). When a stationary textured background was superimposed to the target displacement, the gain of the steady-state eye smooth pursuit velocity was significantly reduced, while the latency of pursuit initiation did not vary significantly, as compared to a dark background condition. The initial velocity of the eye smooth pursuit was also lowered. Both the initial acceleration and the steady-state manual tracking angular velocity were slightly, but not significantly, lowered when compared to a dark background condition. Detrimental effects of the stationary textured background were of comparable amplitude for ocular and manual pursuit. In a second condition, they compared ocular and manual pursuit when the textured background was either stationary or drifting.

Schütz, A. C., Braun, D. I. and Gegenfurtner, K. R., Improved visual sensitivity during smooth pursuit eye movements: Temporal and spatial characteristics, Visual Neuroscience (2009), 26(3), 329-340 describes that they investigated the enhancement over a wide range of temporal and spatial frequencies. In a first Experiment, they measured the temporal impulse response function (TIRF) for colored stimuli. The TIRF for pursuit and fixation differed mostly with respect to the gain but not with respect to the natural temporal frequency. Hence, the sensitivity enhancement seems to be rather independent of the temporal frequency of the stimuli. In a second Experiment, they measured the spatial contrast sensitivity function for luminance-defined Gabor patches with spatial frequencies ranging from 0.2 to 7 cpd.

Spering, M., Kerzel, D., Braun, D. I., Hawken, M. J. and Gegenfurtner, K. R., Effects of contrast on smooth pursuit eye movements, Journal of Vision (2005), 5(5), 455-465 describes that it is well known that moving stimuli can appear to move more slowly when contrast is reduced. In the reference, they address the question whether changes in stimulus contrast also affect smooth pursuit eye movements. Subjects were asked to smoothly track a moving Gabor patch. Targets varied in velocity, spatial frequency, and contrast, ranging from just below individual thresholds to maximum contrast.

Schwob, Noémie and Palmowski-Wolfe, A., Objective measurement of visual acuity by optokinetic nystagmus suppression in children and adult patients, Journal of AAPOS (2019), 23(5), 272.e1-272.e6 describes that to investigate the correlation between subjective and objective visual acuity as elicited with a new computerized optokinetic nystagmus (OKN) suppression test ("SpeedWheel") in adults and school-aged children. Fifteen children (6-12 years) and 27 adults with refractive errors, amblyopia, cataract, agerelated macular degeneration, and thyroid-associated orbitopathy underwent testing of subjective visual acuity with E- and Landolt-C symbols (Freiburg Acuity and Contrast Test [FrACT]) and visual acuity as estimated with the SpeedWheel on an LCD screen.

Schwob, Noemie and Palmowski-Wolfe, A., Establishing an Objective Measurement of Visual Acuity with a Computerised Optokinetic Nystagmus Suppression Test, Klinische Monatsblaetter Fuer Augenheilkunde (2020), 237(4), 502-505 describes their objective investigating the correlation between subjective and objective VA (visual acuity) elicited with a newly developed computerised optokinetic nystagmus (OKN) suppression test ("SpeedWheel") in adults. According to their method, SpeedWheel presented alternating black/white stripes moving horizontally across a LED screen. Seven VA steps were induced with Bangerter filters placed onto spectacle frames. Magnified eye movements were projected from infrared cameras inside the frames and displayed onto a smartphone. Dots whose size increased in logarithmic steps were superimposed to suppress OKN. Suppression of OKN resulted in the SpeedWheel acuity which was then correlated to Snellen acuity as measured with the Freiburg Acuity test.

US 2017/0354369 Al discloses a mobile system for measuring opticokinetic nystagmus in a subject includes a display screen to provide an opticokinetic stimulus and an imaging system to record eye movement data of the subject. The mobile system is configured to compare the stimulus and the recorded eye movement data to provide objective vision acuity testing. The stimulus can include various combinations of motion (e.g., horizontal, vertical, left, right, or other suitable motions), variable rate of movement, variable pattern dimensions (e.g., large-small), and the capacity to change the pattern dimensions (e.g., size, speed, direction) during the course of the test.

WO 2018/006013 Al discloses a system that can measure eye gaze position and detect, in near real-time, smooth eye movements that are driven by a moving stimulus. Smooth movements that match the velocity of a moving stimulus provide evidence that the subject can see the moving stimulus. The present system can give real-time feedback to the user, for example in the form of music, contingent on the ability of the user to perform smooth velocity-matched eye movements. The present system can measure visual impairment and train visual ability both for rehabilitation and development purposes.

WO 2022/015227 Al relates to a device for providing an eye metric, comprising a display unit, producing a visual stimulus to an eye. An eye-tracking unit, measures the eye's movements in response to the stimulus, and an analyzing unit, outputting a metric result. The display unit produces a moving stimulus with at least one varying stimulus parameter such as a symbol size, and the eye-tracking unit detects when the eye loses visual contact with the stimulus. The analyzing unit provides a metric result based on the value of the stimulus parameter at the time when loss of visual contact was detected.

US 2020/0214559 Al discloses a faceguard configured for measuring a human eye muscle movement response. The faceguard is configured for protecting at least one part of a human face and has an aperture for human vision through the faceguard. The faceguard comprises an eye sensor, a head orientation sensor, and an electronic circuit. The eye sensor comprises a video camera and is configured for measuring eyeball movement, pupil size, and/or eyelid movement. The head orientation sensor senses pitch and/or yaw of a person's head. The electronic circuit is responsive to the eye sensor and the head orientation sensor.

US 10 945 599 B1 discloses a system for vision testing and/or training of a user. In one embodiment, the system includes an eye movement tracking device, a visual display device having an output screen, and a data processing device operatively coupled to the eye movement tracking device and the visual display device. The data processing device being programmed to generate and display the at least one visual object on the output screen of the visual display device; displace the at least one visual object across the output screen of the visual display device; and determine, based upon the signal received from the eye movement tracking device, whether the user is able to accurately track the at least one visual object as the at least one visual object is displaced across the output screen of the visual display device.

US 2012/105609A1 discloses a binocular visual performance measuring method including:
a parallax image displaying step of displaying left and right parallax images at a predetermined measurement start position on a display screen when a measurement item is designated; a parallax image changing step of changing one of the left and right parallax images relative to the other of the left and right parallax images in accordance with the designated measurement item; a timing detecting step of detecting a timing when the subject viewing the left and right parallax images from a position a predetermined distance away from the display screen becomes unable to achieve fusion of the left and right parallax images; and a measurement value calculating step of calculating a measurement value of the designated measurement item based on the predetermined distance and a difference between the left and right parallax images defined at the detected timing.

### Problem to be solved

It is therefore an objective of the present invention, in particular in view of US 2020/0214559 Al, to provide a computer-implemented method, a computer program and an apparatus for determining a visual parameter of at least one eye of a person, which at least partially overcome the above-mentioned problems of the state of the art.

It is a particular objective of the present invention to provide an easy, precise, fast and still reliable approach to determine the at least one visual parameter of the at least one eye of the person.

### Summary of the invention

This problem is solved by a computer-implemented method, a computer program and an apparatus for determining a visual parameter of at least one eye of a person with the features of the independent claims. Preferred embodiments, are listed in the dependent claims or throughout the following description. The invention is set out in the appended set of claims.

In a first aspect, the present invention relates to a computer-implemented method for determining at least one visual parameter of at least one eye of a person, the method comprising the following steps:
a) displaying on a screen to at least one eye of a person at least one first visual stimulus, wherein at least a portion of the at least one first visual stimulus has a first moving spatial location; and
b) displaying on the screen to the at least one eye of the person at least one second visual stimulus, wherein at least a portion of the at least one second visual stimulus has a second moving spatial location;
   wherein the at least one first visual stimulus and the at least one second visual stimulus are displayed at the same time on the screen effecting a resulting eye movement depending on the at least one visual parameter;
c) generating tracking data about the resulting eye movement of the at least one eye of the person by using at least one eye-tracking device; and
d) determining at least one visual parameter of the at least one eye of the person by comparing the tracking data, the first moving spatial location and the second moving spatial location by using at least one processing device;

wherein the at least one first visual stimulus is a pursuit stimulus and the at least one second visual stimulus is a pursuit stimulus,
wherein the spatial location of the at least one first visual stimulus and the spatial location of the at least one second visual stimulus are coinciding at a coinciding spatial location, wherein the at least one first visual stimulus and the at least one second visual stimulus move, starting from the coinciding spatial location, in such a manner that the spatial location of the at least one first visual stimulus and the spatial location of the at least one second visual stimulus are no longer coinciding,
wherein the coinciding spatial location is a matching spatial location, wherein at this matching spatial location at least a portion of the at least one first visual stimulus covers up at least a portion of the at least one second visual stimulus or vice versa in such a manner that the covered portion of the at least one second visual stimulus or the covered portion of the at least one first visual stimulus is not perceptible by the at least one eye of the person,
wherein the at least one first visual stimulus and the at least one second visual stimulus move, starting from the coinciding spatial location, with at least one of a spatial movement direction or a spatial movement speed being different from each other, thereby, the at least one first visual stimulus and the at least one second visual stimulus move away from the coinciding position and/or away from one another,
wherein the at least one first visual stimulus and the at least one second visual stimulus are different in at least one further visual stimulus parameter selected from at least one of: a spatial frequency, a spatial frequency range, or a contrast level.

As generally used, the term "computer-implemented method" refers to a method which involves a programmable apparatus, in particular, a computer, a computer network, or a readable medium carrying a program, whereby at least one of the steps of the method, specifically all steps, are performed by using at least one computer program. Alternatively, the at least one computer program may be accessible by an apparatus which may be adapted for performing the method via a network, such as via an in-house network or via internet. With particular regard to the present invention, the present method can, thus, be performed on a programmable apparatus which is configured for this purpose, such as by providing a computer program which is configured for such a purpose.

As generally used, the term "determining" or any grammatical variation thereof refers to a process of generating at least one representative result. With particular regard to the present invention, the at least one result comprises information about the at least one visual parameter of the at least one eye of the person.

As used herein, the term "visual parameter" refers to a refractive error and/or a visual performance of the at least one eye of the person. As generally used, the terms "refraction" or "refractive" refer to a bending of incident light entering the interior of the eye of the person via the pupil, wherein the term "refractive error" refers to an observation that the incident light may, in particular owing to a form of the eye, not be focusing appropriately on the retina of the eye, resulting in a defocus of the eye. As used herein, the term "visual performance" refers to a characteristic that is indirectly and/or directly related to the at least one eye of the person, wherein the visual performance may be determined by investigating the at least one eye of the person by using an adapted measurement procedure.

According to step a), at least one first visual stimulus is displayed on a screen to at least one eye of a person, wherein at least a portion of the at least one first visual stimulus has a first moving spatial location. Therefore, the at least one first visual stimulus is presented to the at least one eye of the person. At least one portion of a display area of the at least one first visual stimulus is moving. This movement may elicit an eye movement.

As generally used, the term "displaying" or any grammatical deviation thereof refers to a presentation of at least one of an image, an item, a text, or a video, particularly at least the at least one first visual stimulus and/or the at least one second visual stimulus, on the at least one screen. As generally used, the term "screen" refers to an electronic visual display device designated for the presentation of at least one of an image, an item, text, or a video transmitted electronically. With particular regard to the present invention, the screen may be configured for displaying the at least one first visual stimulus to the at least one eye of a person, particularly in such manner that the at least one first visual stimulus may be perceptible by the at least one eye of the person.

As used herein, the term "visual stimulus" refers to a graphical presentation of an item, which is known or reasonably to be expected by the person skilled in the art to elicit the at least one eye movement in the at least one eye of the person. As used herein, the term "portion" refers to a part of the graphical presentation of the visual stimulus. As used herein, the term "spatial location" refers to specific position of the at least one respective visual stimulus on the screen. As used herein, the term "moving", or any grammatical variation, thereof refers to a change in the spatial location. The moving spatial location is a time-varying spatial location having a spatial movement speed and a spatial movement direction.

As used herein, the terms "first" or "second" or "third" are considered as a description of an element without specifying an order or a chronological sequence and without excluding a possibility that other elements of the same may be present. A "first" element may be different from a "second" element and a "third" element. This holds for any possible permutation.

According to step b), at least one second visual stimulus is displayed on the screen to the at least one eye of the person, wherein at least a portion of the at least one second visual stimulus has a second moving spatial location. Therefore, the at least one second visual stimulus is presented to the at least one eye of the person. At least one portion of a display area of the at least one second visual stimulus is moving. This movement may elicit an eye movement. With particular regard to the present invention, the screen may be configured for displaying the at least one second visual stimulus to the at least one eye of a person, particularly in such manner that the at least one second visual stimulus may be perceptible by the at least one eye of the person.

Further according to the first aspect, the at least one first visual stimulus and the at least one second visual stimulus are displayed at the same time on the screen, thereby effecting a resulting eye movement depending on the at least one visual parameter.

As used herein, the term "at the same time" refers to displaying the at least one first visual stimulus at a first display time and the at least one second visual stimulus at a second display time, wherein the first display time and the second display time overlap at least one of: partially; or fully. As used herein, the term "display time" refers to a period of time during which the at least one first visual stimulus and/or the least one second visual stimulus are displayed on the screen.

As used herein, the term "effecting" refers to the at least one first visual stimulus and/or the at least one second visual stimulus causing the resulting eye movement. Therefore, the resulting eye movement may result from the at least one first visual stimulus, the at least one second visual stimulus or a combination of the at least one first visual stimulus and the at least one second visual stimulus.

According to step c), tracking data about the resulting eye movement of the at least one eye of the person are generated by using at least one eye-tracking device. The eye-tracking device may be configured to record the resulting eye movement.

As generally used, the term "tracking" or any grammatical deviation thereof refers to recording motions of the at least one eye by using the at least one eye-tracking device. As generally used, the term "eye-tracking device" refers to a device that is used to record the motion of the at least one eye of the person, particularly to record a change of the line of sight and/or a gaze position of the at least one eye of the person. As a result of the recording, eye tracking data comprising information about the motion of the at least one eye of the person is generated, wherein the information about the motion of the at least one eye of the person may be given by the time-variance of the line of sight and/or the gaze position of the at least one eye. At least one outcome may be provided comprising the tracking data. Based on standard ISO 13666:2019, Section 3.2.24, the term "line of sight" refers to a path from a point of interest, i.e. a point of fixation, in object space to a center of an entrance pupil of the eye of the person and, further, comprise a continuation in image space from a center of an exit pupil to a retinal point of fixation, generally the foveola, in the eye of the person. Standard ISO 13666:2019 will in the following also be refered to as "Standard".

In a particularly preferred embodiment, the term "at the same time" may refer to displaying the at least one first visual stimulus at a first display time and the at least one second visual stimulus at a second display time, wherein the first display time and the second display time overlap at least one of: partially; or fully. As used herein, the term "partially" refers to an overlap of the first display time and the second display time, wherein only a portion of the first display time overlaps with the second display time; or vice versa. As used herein, the term "fully" refers to an overlap of the first display time and the second display time, wherein the complete first display time overlaps the complete second display time.

In a particularly preferred embodiment, the at least one first visual stimulus and the at least one second visual stimulus may be perceptible from the at least one eye of the person in a manner that the resulting eye movement is generated by at least one of: the at least one first visual stimulus;or the at least one second visual stimulus. In a particularly preferred embodiment, the at least one first visual stimulus and the at least one second visual stimulus may cause the resulting eye movement depending on the at least one visual parameter of the at least one eye of the person to be determined.

In a particularly preferred embodiment, the at least one first visual stimulus and/or the at least one second visual stimulus may be displayed in such a manner that the at least one first visual stimulus and/or the at least one second visual stimulus are visible to the at least one eye of the person at the same time, particularly visible to at least one of: a central field of view of the at least one eye of the person: or a peripheral field of view of the at least one eye of the person. The term "central field of view" refers to a portion of the field of view comprising the line of sight. The central field of view is surrounded by the peripheral field of view, particularly directly surrounded. The term "peripheral field of view" is a portion of the field of view that comprises a vision occurring outside the gaze position. The line of sight is not comprised in the peripheral field of view. The peripheral field of view is outside of a central field of view.

In a particularly preferred embodiment, the at least one first visual stimulus may be visible to at least one of: the central field of view of the at least one eye of the person; or the peripheral field of view of the at least one eye of the person. In a particularly preferred embodiment, the at least one second visual stimulus may be visible to at least one of: the central field of view of the at least one eye of the person; or the peripheral field of view of the at least one eye of the person. In a particularly preferred embodiment, at least one of the first visual stimulus; or the at least one second visual stimulus may be appearing, particularly in at least one of: the central field of view of the at least one eye of the person; or the peripheral field of view of the at least one eye of the person unexpected by the person. Therefore, the line of sight can be assumed to intersect with the at least one first visual stimulus or the at least one second visual stimulus, particularly a center of the at least one first visual stimulus or a center of the at least one second visual stimulus. As used herein, the term "appearing" refers to starting the displaying of the at least one first visual stimulus and/or the at least one second visual stimulus, particularly in the central field and/or the peripheral field, respectively. In a particularly preferred embodiment, a central field of view opening angle α may be smaller than at least one of: 2°, 3°; 4°; 5°; 6 °; or 8°.

In a particularly preferred embodiment, step c) my be performend during step a) and step b). In a particularly preferred embodiment, step a) and step b) may be performed at the same time. In a particularly preferred embodiment, steps a) to c) may define a measurement cycle. The term "measurement cycle" refers herein to a sequence of at least the steps a), b) and c), wherein step d) may, additionally, be comprised by the measurement cycle. Additionally, a step e), step f), step g) and/or step h) may be comprised in the measurement cycle. In a particularly preferred embodiment, at least 2; at leat 5; at least 10; at least 50; at least 100 measurement cylces may be performed.

According to step d), at least one visual parameter of the at least one eye of the person is determine by comparing the tracking data, the first moving spatial location and the second moving spatial location by using at least one processing device. The processing device may be configured to determine the at least one visual parameter of the at least one eye of the person.

As used herein, the term "comparing" refers to analyzing a first information in the light of a second information, particularly the information contained in the tracking data in the light of the first moving spatial location and the second moving spatial location. As generally used, the term "processing device" refers to at least one component configured for processing the information. The at least one component may be selected from at least one of: a CPU, a memory and a motherboard.

In a particularly preferred embodiment, the at least one visual parameter of the at least one eye of the person may be selected from at least one of a refractive error or a visual performance of the at least one eye of the person. In a particularly preferred embodiment, the refractive error of the at least one eye of the person may be at least one of a value related to:
- a spherical power;
- a cylindrical power;
- a cylinder axis; or
- an addition.

The determination of the refractive error may be of particular interest for the present invention. As defined in the Standard, Section 3.12.2, the term "spherical power", usually abbreviated to "sphere" or "sph", refers to a value of a back vertex power of a spherical-power lens, or for a back vertex power in one of two principal meridians of an astigmatic-power lens, depending on a principal meridian chosen for reference. The spherical power of the at least one eye of the person may be a value related to a "spherical equivalent". As defined in the Standard, Section 3.13.7, the term "cylindrical power", usually abbreviated to "cylinder" or "cyl", refers to an algebraic difference between principal powers with power of the principal meridian chosen for reference being subtracted from the other principal power. As defined in the Standard, Section 3.13.8, the term "cylinder axis", usually abbreviated to "cyl axis" or "axis", refers to a direction of the principal meridian of a lens whose vertex power is chosen for reference. As defined in the Standard, Section 3.16.3, the term "addition", also abbreviated to "add", refers to a difference between the vertex power of a near portion and the vertex power of a distance portion in a multifocal or power-variation lens.

In a particularly preferred embodiment, the visual performance may be selected from at least one of
- a visual acuity, particularly selected from at least one of:
   ∘ a near field visual acuity; or
   ∘ a far field visual acuity;
- a contrast sensitivity;
- a color vision; or
- a visual field.

As generally used, the term "visual acuity" refers to a spatial resolution ability of the at least one eye of the person with respect to a structure within at least one visual target. As generally used, the "near field" refers to a distance of up to 40 centimeters, and preferably of at least 25 centimeters. As generally used, the "far field" refers to a distance of at least 5 meters. As further generally used, the term "contrast sensitivity" refers to a property of at least one eye of a person to discern between different luminance levels in at least one visual target. As further generally used, the term "color vision" refers to a property of the at least one eye of the person to discern between different colors comprised by at least one visual target. As generally used, the term "visual field" refers to a spatial area which is perceptible by the at least one eye of the person. The visual field comprises the central field of view and the peripheral field of view.

Further according to the invention, the at least one first visual stimulus and the at least one second visual stimulus are a pursuit stimulus. As used herein, the term "pursuit stimulus" refers to a visual stimulus designated to elicit a pursuit eye movement. The displaying of a pursuit stimulus may be recognized by the person as a task, in particular to follow the pursuit stimulus and, thereby, to elicit the eye movement. In case a plurality of pursuit stimuli is displayed, the task may be recognized by the person as to follow one of the pursuit stimuli. However, an optokinetic nystagmus stimulus may, in general, not be recognized by the person to provide such a task. Each visual stimulus may elicit an eye movement, particularly, the at least one first visual stimulus may elicit a first eye movement while the at least one second visual stimulus may elicit a second eye movement. In accordance with the present invention, at least one of the eye movements, particularly the first eye movement and/or the second eye movement, is a pursuit eye movement, particularly being a conscious eye movement, while at least one further of the eye movements may, preferably, be an optokinetic nystagmus, particularly being a reflexive eye movement in the at least one eye of the person. As generally used, the term "optokinetic nystagmus" refers to an eye movement that comprises a slow phase and a quick phase, wherein the slow phase comprises a pursuit eye movement and the quick phase a saccadic eye movement. As generally used, the term "pursuit eye movement" refers to an eye movement in which the at least one eye of the person remains fixated on at least a portion of a visual stimulus with a moving spatial location, particularly a moving center of a visual stimulus. As generally used, the term "saccadic eye movement" refers to an eye movement that comprises a quick movement of the at least one eye of the person between at least two phases of fixation. As used herein, the term "reflexive" refers to an eye movement being unintended by the person. As used herein, the term "conscious" refers to an eye movement being intended by the person.

The pursuit stimulus is designated to elicit a pursuit eye movement. In a particularly preferred embodiment, the pursuit stimulus may be selected from at least one of:
- a Gabor patch;
- a noise patch, particularly having a predefined spatial frequency;
- a circle;
- a ring structure, particularly a ring structure having a plurality of rings having a defined radial spatial frequency;
- a grid, particularly a grid comprising Gabor patches of different tilts and/or spatial frequencies;
- a star; or
- a letter, particularly selected from at least one of:
   ∘ a tumbling E; or
   ∘ a Landolt C.

As used herein, the term "different" refers to two items being dissimilar. As gernerally used, the term "tumbling E" and "Landolt C" each refers to a standardized symbol used for testing vision.

As further used herein, the term "optokinetic nystagmus stimulus" refers to a visual stimulus designated to elicit an optokinetic nystagmus. In a particularly preferred embodiment, the optokinetic nystagmus stimulus may be designated to elicit an optokinetic nystagmus. In a particularly preferred embodiment, the optokinetic nystagmus stimulus may have a structured appearance. As used herein, the term "appearance" refers to a look of the respective stimulus, particularly the at least one first visual stimulus or the at least one second visual stimulus. As used herein, the term "structure", or any grammatical variation thereof, refers to at least one visible first portion of the visual stimulus having an appearance and/or look being different from at least one second portion of the same visual stimulus. In a particularly preferred embodiment, the structured appearance may be shifting, particularly translationally shifting, in at least one direction. As used herein the term "shifting" refers to a movement of a portion of the visual stimulus, particularly the first portion and/or the second portion. As used herein, the term "translational" refers to a movement wherein each point of the moved object undergos the same movement, particularly wherein the object is a portion of the visual stimulus, more particularly wherein the object is the first portion and/or the second portion. In a particularly preferred embodiment, the structured appearance may be described by at least one spatial frequency. As further generally used, the term "spatial frequency" refers to a reciprocal value of a spatial distance reflecting a spatial period of repetition in the at least one visual stimulus.

In a particularly preferred embodiment, the optokinetic nystagmus may comprise a slow phase and a quick phase, wherein in the slow phase the pursuit eye movement is elicited by the at least one spatial frequency, and wherein in the quick phase a saccadic eye movement is a reset movement of the at least one eye.

In a particularly preferred embodiment, the optokinetic nystagmus stimulus may be selected from at least one of:
- a Gabor patch; or
- a noise patch;
specifically having
- a sinusoidal pattern; or
- a stripe pattern.

The term "Gabor patch" refers to gratings, usually with a Gaussian envelope, which are known to be particularly useful as visual stimulus for the person's eye. As generally used, the term "noise" refers to an interference quantity with a broad non-specific frequency spectrum. The noise patch is the visual presentation of this noise. A noise patch, particularly when used as an optokinetic nystagmus stimulus may further show at least one defined spatial frequency. As used herein, the term "pattern" refers to a structure having a portion that is repeated, particularly in a constant manner. As used herein, the term "sinusoidal" refers to an appearance and repetition of the repeated portion in a sinusoidial manner. As used herein, the term "stripe" refers to an appearance and repetition of the repeated portion in a stripe manner.

In a particularly preferred embodiment, the at least one visual stimulus parameter of the optokinetic nystagmus stimulus may be selected from at least one of:
- a first spatial frequency eliciting the optokinetic nystagmus in a first direction; or
- a second spatial frequency eliciting the optokinetic nystagmus in a second direction.

In a particularly preferred embodiment, the first direction of the optokinetic nystagmus elicited by the first spatial frequency and the second direction of the optokinetic nystagmus elicited by the second spatial frequency may differ from each other. In a particularly preferred embodiment, the first direction of the optokinetic nystagmus may be elicted, whereas the second direction of the optokinetic nystagmus may be elicited in succession. As used here, the term "succession" refers to the first direction is being elicited before the second direction is being elicited, particularly the second direction is being elicited after eliciting of the first direction is stopped.

In a particularly preferred embodiment, the at least one visual stimulus parameter of the optokinetic nystagmus stimulus may, alternatively or in addition, be:
- a third spatial frequency eliciting the optokinetic nystagmus in a third direction.

In a particularly preferred embodiment, the third direction of the optokinetic nystagmus elicited by the third spatial frequency mnay differ from the first direction of the optokinetic nystagmus elicited by the first spatial frequency and the second direction of the optokinetic nystagmus elicited by the second spatial frequency.

In a particularly preferred embodiment, at least one of: the at least one first visual stimulus; the at least one second visual stimulus, may be blurred. As generally used, the term "blurring" or any grammatical variation thereof refers to a process in which an image, particularly the graphical presentation of the visual stimulus, is smoothend.

In a particularly preferred embodiment, a spatial location, particularly of a center, of the pursuit stimulus may be time varying, particularly, when the spatial movement speed is not 0. As used herein, the term "time varying" refers to the spatial location changing over time. Meaning that a spatial location of the pursuit stimulus is different at a first time from a spatial location of the pursuit stimulus at a second time. In a particularly preferred embodiment, an appearance of the pursuit stimulus may be maintained, particularly wherein the appearance has no translational shift. As used herein, the term "maintaining" or any grammatical variation thereof refers to the appearance not changing over time but being constant. Meaning that an appearance of the pursuit stimulus is the same at a first time and stimulus at a second time.

In a particularly preferred embodiment, the at least one first visual stimulus may be defined by using at least one first visual stimulus parameter, and wherein the at least one second visual stimulus is defined by using at least one second visual stimulus parameter, particularly wherein the first visual stimulus parameter and the second visual stimulus parameter are further compared for determining the at least one visual parameter. In a particularly preferred embodiment, the at least one first visual stimulus parameter and the at least one second visual stimulus parameter may differ from each other, particularly by being different paramenters or having a different value for the same paramenter.

As used herein, the term "display area" refers to a field on the screen on which an item, particularly the respective visual stimulus, is displayed. As used herein, the term "display time" refers to a time period in which an item, particularly the respective visual stimulus, is displayed. As used herein, the term "spatial movement speed" refers to a velocity of the respective visual stimulus, particularly of the center of the respective visual stimulus. As used herein, the term "spatial movement direction" refers to a heading of the respective visual stimulus, particularly of the center of the respective visual stimulus. As further generally used, the term "spatial frequency range" refers to a range of several reciprocal values of a spatial distance reflecting a spatial period of repetition in the at least one visual stimulus. As generally used, the term "contrast" refers to a luminance level in the at least one visual stimulus.

In a particularly preferred embodiment, at least one of:
- the at least one first visual stimulus parameter; or
- the at least one second visual stimulus parameter
may be varied over time, specifically
- in a continuous manner, particularly in a monotonous manner; or
- in a stepwise manner.

As used herein, the term "continuous" means that the visual stimulus parameter attributed to the appearance is varied perpetual and/or ongoing. The term "monotonous" refers to the visual stimulus parameter attributed to the appearance being varied uniformly and/or in a steady manner. In other words, a change in the visual stimulus parameter may be not time-varying but be maintained.

In a particularly preferred embodiment, the at least one first visual stimulus parameter and the at least one second visual stimulus parameter may be varied in succession, particularly wherein the at least one first visual stimulus parameter is varied when the at least one second visual stimulus parameter is mainained constant, or vice versa. As used here, the term "succession" refers to the at least one first visual stimulus parameter is varied before the at least one second visual stimulus parameter is varried.

In a particularly preferred embodiment, a plurality of the at least one first visual stimulus may be displayed during step a). In a particularly preferred embodiment, a plurality of the at least one second visual stimulus may be displayed during step b). As used herein, the term "plurality" refers to a quantity of at least two units, preferably more than two units, particularly a quantity of at least two pieces, preferably of more than two pieces. In a particularly preferred embodiment, at least two and/or all visual stimuli of the plurality of at least one first visual stimulus displayed during step a) may be the same visual stimulus; and/or at least two and/or all visual stimuli of the plurality of at least one second visual stimulus displayed during step b) are the same visual stimulus.

In a particularly preferred embodiment, the at least one first visual stimulus or the at least one second visual stimulus may perform a transition from a visual stimulus designated for eliciting a given eye movement to a visual stimulus designated for eliciting a different eye movement. As used herein, the term "transition" refers to a change of the respective stimulus designated for eliciting a first eye movement to a visual stimulus designated for eliciting a second eye movement. In a particularly preferred embodiment, the at least one of the at least one first visual stimulus or the at least one second visual stimulus may perform the transition from a pursuit stimulus to an optokinetic nystagmus stimulus; or vice versa, wherein at least one of: the at least one first visual stimulus; or the at least one second visual stimulus may still be the pursuit stimulus.

In a particularly preferred embodiment, at least one third visual stimulus may be displayed on the screen to the at least one eye of the person, particularly displayed during at least of of:
- step a); or
- step b).

In a particularly preferred embodiment, the at least one third visual stimulus may be a noise patch. In a particularly preferred embodiment, the noise of the noise patch may be selected from at least one of:
- a static noise;
- a spatial frequency filtered noise;
- a dynamic noise; or
- a dynamic spatial frequency filtered noise.

In a particularly preferred embodiment, the noise patch may be performing a translational movement. As used herein, the term "static noise" refers to a noise having an appearance which is time invariant. As used herein, the term "dynamic noise" refers to a noise having an appearance which is varying over time. The at least one third visual stimulus may cover the screen completely.

In a particularly preferred embodiment, a display area of the at least one first visual stimulus may be larger than a display area of the at least one second visual stimulus, particularly during a complete measurement cycle. In a particularly preferred embodiment, the display area of the at least one first visual stimulus may comprise at least partially, preferably completely, the display area of the at least one second visual stimulus, particularly during a complete measurement cycle.

According to the invention, the at least one first visual stimulus is a pursuit stimulus; and the at least one second visual stimulus is a pursuit stimulus. According to the invention, the spatial location, particularly of the center, of the at least one first visual stimulus and the spatial location, particularly of the center, of the at least one second visual stimulus are coinciding at a coinciding spatial location. As used herein, the term "center" refers to the midpoint of the display area of the respective stimulus. As used herein, the term "coinciding" refers to matching spatial location. At this matching spatial location at least a portion of the at least one first visual stimulus covers up at least a portion of the at least one second visual stimulus in such a way that the covered portion of the at least one second visual stimulus is not perceptible by the at least one eye of the person, or vice versa. At least a portion of the at least one first visual stimulus may be transparent in such a way that at least a portion of the at least one second visual stimulus may still be perceptible by the at least one eye of the person, or vice versa. In a particularly preferred embodiment, the coinciding spatial location may be moving.

According to the invention, the at least one first visual stimulus and the at least one second visual stimulus move, starting from the coinciding spatial location, in such a manner that the spatial location, particularly of the center, of the at least one first visual stimulus and the spatial location, particularly of the center, of the at least one second visual stimulus are no longer coinciding. As used herein, the term "no longer" refers to a change of a status given in a time-related past that is no longer present in a time-related present. Specifically, when the spatial locations are no longer coinciding, the at least one first visual stimulus may change its status in the time-related past from covering up at least the portion of the at least one second visual stimulus in such a way that the covered portion of the at least one second visual stimulus is not perceptible by the at least one eye of the person, or vice versa. The covered portion may therefore in the time-related present be perceptible by the at least one eye of the person. A spatial location that is no longer coincinding may be separating.

According to the invention, the at least one first visual stimulus and the at least one second visual stimulus move, starting from the coinciding spatial location, with at least one of:
- a spatial movement direction; or
- a spatial movement speed being different from each other. Thereby, the at least one first visual stimulus and the at least one second visual stimulus move away from the coinciding position and/or away from one another. According to the invention, the at least one first visual stimulus and the at least one second visual stimulus are different in at least one further visual stimulus parameter, at least one further visual stimulus parameter being selected from at least one of:
   - a spatial frequency;
   - a spatial frequency range; or
   - a contrast level.

In a particularly preferred embodiment, generating the tracking data may further comprise recording a time stamp at which the at least one resulting eye movement occurs for at least one of: the first time; or the last time. As used herein, the term "time stamp" refers to a defined point in time. As used herein, the term "first time" refers to a specific event taking place that has not been taking place before. As used herein, the term "last time" refers to the specific event no longer taking place, wherein the specific event has been taking place before.

In a particularly preferred embodiment, the method may further be comprising a step of:
e) recording at least one distance between the at least one eye of the person and the screen displaying at least one of the at least one first visual stimulus or the at least one second visual stimulus.

As used herein, the term "distance" refers to a length of a line that connects two points, wherein the line represents the shortest connection of the two points. The first point may be the at least one eye and the second point may be the at least one first visual stimulus or the at least one second visual stimulus.

In a particularly preferred embodiment, the method may further be comprising a step of:
f) recording at least one line of sight of the at least one eye of the person. For the term "line of sight", reference can be made to the Standard, Section 3.2.24, as already indicated above.

In a particularly preferred embodiment, the method may further be comprising a step of:
g) recording at least one gaze position of the at least one eye of the person. As used herein, the term "gaze position" refers to a point at which at least one object and the at least one line of sight of the at least one eye of the person intersect.

In a particularly preferred embodiment, the method may further be comprising a step of:
h) recording a head movement of the head of the person comprising the at least one eye. As generally used, the term "head movement" refers to a movement of the head of the person, which may be dependent or independent from the at least one resulting eye movement depending on the at least one visual parameter. The head movement and resulting eye movement may both influence the line of sight and/or the gaze position of the at least one eye of the person.

In a particularly preferred embodiment, an indication of at least one of:
- the at least one visual stimulus parameter of the at least one first visual stimulus; or
- the at least one visual stimulus parameter the at least one second visual stimulus may be requested from the person. As used herein, the term "indication" refers to a detail or an information about at least one item or object, specifically the at least one first visual stimulus and/or the at least one second visual stimulus. As used herein, the term "request" refers to an inquiry of the person.

In a particularly preferred embodiment, the request may be at least one of:
- a visual prompt;
- an auditive prompt; or
- a tactile prompt.

As used herein, the term "visual prompt" refers to a request that is visually perceptible by the person. Such a request may be a question of which the lettering is being displayed on the screen. As used herein, the term "auditive prompt" refers to a request that is audibly perceptible by the person. Such a request may be a question that is played on a speaker. As used herein, the term "tactile prompt" refers to a request that is tactilely perceptible by the person. Such a request may be a vibration iniciated by a vibration generator, particularly of a smartphone.

**In** a particularly preferred embodiment, determining the at least one visual parameter of the at least one eye of the person may comprise analyzing at least one outcome. As generally used, the term "analyzing" refers to a systematic investigation in which the at least one outcome under investigation is broken down into its components. These components are thereby recorded on the basis of criteria and subsequently ordered, examined and evaluated. In a particularly preferred embodiment, the at least one outcome may comprise:
- the tracking data about the at least one resulting eye movement, particularly selected from at least one of:
   ∘ the at least one gaze position of the at least one eye of the person; or
   ∘ the at least one line of sight of the at least one eye of the person; and
- the first moving spatial location and the second moving spatial location, particularly selected from at least one of:
   ∘ the at least one first visual stimulus;
   ∘ the at least one second visual stimulus; and
wherein at least one outcome further comprises at least one of:
- the at least one visual stimulus parameter of the at least one first visual stimulus; or
- the at least one visual stimulus parameter of the at least one second visual stimulus.

In a particularly preferred embodiment, the at least one outcome may further comprise at least one of:
- the tracking data about the at least one head movement of the head of the person; or
- the at least one distance between the at least one eye of the person and the screen displaying at least one of the at least one first visual stimulus or the at least one second visual stimulus.

In a particularly preferred embodiment, determining the visual parameter by analyzing the outcome may be performed by using at least one of:
- an analytical method;
- a regression method;
- a statistical analysis, particularly a Multivariate statistic analysis, more particularly a Principle Component analysis; or
- a machine learning algorithm.

As gernerally used, the term "analytical" refers to a method that is based on at least one mathematical function. As generally used, the term "regression" refers to a statistical analysis tool that aims to determine a relationship between an input data and a statistical model to determine output parameters. In this process, the statistical model may be fitted onto the input data. As generally used, the term "statistical analysis" refers to an interpretation of the outcome in order to uncover patterns and trends. As generally used, the term "Multivariate statistic analysis" refers to a simultaneous analysis of more than one outcome variable. As gernerally used, the term "Principle Component analysis" refers to an orthogonal linear transformation that transforms the outcome to a new coordinate system such that the greatest variance by some scalar projection of the outcome comes to lie on the first corrdinate, the second greatest variance on the second coordinate, and so on. As generally used, the term "machine learning algorithm" refers to a process of applying artificial intelligence to automatically generate a statistical model. A machine learning algorithm configured to generate the desired model based on a large number of training data sets can, preferably, be used.

In a particularly preferred embodiment, the method may further be comprising a step of training of the machine learning algorithm for determining of the visual parameter by
- providing training data, comprising
   ∘ tracking data about the at least one resulting eye movement;
   ∘ stimulus data about at least one first visual stimulus and the at least one second visual stimulus, particularly the spatial location of the at least one first visual stimulus and the spatial location of the at least one second visual stimulus;
   ∘ known data about the visual parameter;
- determining preliminary data about the visual parameter from the tracking data and the stimulus data;
- determining a deviation between the preliminary data about to the visual parameter and the known data about the visual parameter; and
- adjusting the machine learning algorithm intended for minimizing the deviation;
wherein the step of training is repeated until the deviation is below a threshold.

As generally used, the term "training" or grammatical variations thereof refers to a process of building a trained model, in particular determining parameters, in particular weighs, of the model. The training may comprise at least one optimization or tuning process, wherein a best parameter combination is determined. The term "training data" refers to a data set on which the machine and deep learning model is trained. The term "threshold" refers to a maximal deviation.

In a particularly preferred embodiment, analyzing the tracking data may comprise analyzing the at least one resulting eye movement in the slow phase. In a particularly preferred embodiment, analyzing the at least one resulting eye movement in the slow phase may comprise determining a velocity of the eye movement. In a particularly preferred embodiment, analyzing the tracking data may comprise analyzing at least one of:
- a latency;
- an acceleration; or
- a velocity
of the at least one eye movement.

As generally used, the term "latency" refers to a time difference between displaying an visual stimulus intended for eliciting an eye movement and the occurring of the intended eye movement of the at least one eye of the person. As generally used, the term "acceleration" refers to a speeding up of the eye movement, particularly the intended eye movement. As gernerally used, the term "velocity" refers to an eye movement speed of the at least one eye of the person, particularly the intended eye movement.

According to a further aspect, the present invention relates to a computer program comprising instructions which, when the program is executed by a computer, cause the computer to carry out the method according to the first aspect or any one of the preceding preferred embodiments. For this purpose, the computer program may comprise instructions provided by means of a computer program code which are capable of performing all of the steps of the methods according to the present invention when implemented on a computer, a data processing device or an apparatus for determining a visual parameter of at least one eye of a person. The computer program code may be provided on a data storage medium or a separate device such as an optical storage medium, e.g. on a compact disc, directly on a computer or data processing device, or via a network, such as via an in-house network or via internet. For further details concerning the computer program, reference may be made to the methods according to the present invention as disclosed elsewhere herein.

According to a further aspect, the present invention relates to an apparatus for determining a visual parameter of at least one eye of a person, the apparatus comprising:
- at least one screen, wherein the at least one screen is configured for displaying to the at least one eye of a person
   ∘ at least one first visual stimulus, wherein at least a portion of the at least one first visual stimulus has a first moving spatial location; and
   ∘ at least one second visual stimulus, wherein at least a portion of the at least one second visual stimulus has a second moving spatial location;
   ∘ wherein the at least one first visual stimulus and the at least one second visual stimulus are displayed at the same time on the screen effecting a resulting eye movement depending on the at least one visual parameter;
- at least one eye-tracking device, wherein the at least one eye-tracking device is configured for generating tracking data about the resulting eye movement of the at least one eye of the person; and
- at least one processing device, wherein the at least one processing device is configured for determining at least one visual parameter of the at least one eye of the person by comparing the tracking data, the first moving spatial location and the second moving spatial location,
wherein the at least one first visual stimulus is a pursuit stimulus and the at least one second visual stimulus is a pursuit stimulus, wherein the spatial location of the at least one first visual stimulus and the spatial location of the at least one second visual stimulus are coinciding at a coinciding spatial location, wherein the at least one first visual stimulus and the at least one second visual stimulus move, starting from the coinciding spatial location, in such a manner that the spatial location of the at least one first visual stimulus and the spatial location of the at least one second visual stimulus are no longer coinciding, wherein the coinciding spatial location is a matching spatial location, wherein at this matching spatial location at least a portion of the at least one first visual stimulus covers up at least a portion of the at least one second visual stimulus or vice versa in such a manner that the covered portion of the at least one second visual stimulus or the covered portion of the at least one first visual stimulus is not perceptible by the at least one eye of the person, wherein the at least one first visual stimulus and the at least one second visual stimulus move, starting from the coinciding spatial location, with at least one of a spatial movement direction or a spatial movement speed being different from each other, thereby, the at least one first visual stimulus and the at least one second visual stimulus move away from the coinciding position and/or away from one another, wherein the at least one first visual stimulus and the at least one second visual stimulus are different in at least one further visual stimulus parameter selected from at least one of: a spatial frequency, a spatial frequency range, or a contrast level.

In a particularly preferred embodiment, the screen and the eye-tracking device may be comprised by at least one of:
- a virtual reality headset;
- an augmented reality system;
- a desktop computer;
- a television set;
- smart glasses; or
- a mobile communication device,
and wherein the processing device is comprised by at least one of
- the virtual really headset;
- the augmented reality system;
- the desktop computer;
- the television set;
- the smart glasses; or
- the mobile communication device.

As generally used, the term "virtual reality headset" refers to a head-mounted device that provides virtual reality for the wearer. As generally used, the term "augmented reality overlay device" refers to a hardware for an interactive experience between a real-world environment and computer-generated perceptual information. As generally used, the term "desktop computer" refers to a computer in a housing shape suitable for use as a workstation computer on desks. As generally used, the term "television set" refers to a device having a tuner, a display and at least one loudspeaker for a purpose of viewing and listening to television broadcasting through at least one of satellite or cable, wherein the television set may also be used as a monitor. As generally used, the term "smart glasses" refers to wearable spectacles having computer functionality and may have connectivity. They may add information perceptible for the at least one eye of the person. As generally used, the term "mobile communication device" refers to a portable wireless telecommunications equipment that may transmit and/or receive voice, video, or computer data.

In a particularly preferred embodiment, the mobile communication device may be selected from at least one of:
- a smartphone;
- a tablet; or
- a laptop.

As generally used, the term "smartphone" refers to a mobile phone having extensive computer functionalities and connectivity. As generally used, the term "tablet" refers to a portable, flat touch-screen computer. As generally used, the term "laptop" refers to a special type of computer having a screen movably attached to a housing, wherein the screen may be folded onto the housing.

In a particularly preferred embodiment, the at least one eye-tracking device may be selected from at least one of:
- a camera, particularly at least one of a front camera and/or a back camera of the smartphone;
- a webcam;
- eye tracking glasses; or
- a visually evoked potential device.

As generally used, the term "camera" refers to an optical device that captures visual images. As generally used, the term "webcam" refers to a small camera that may sit on a monitor, or is built into a computer. As generally used, the term "eye tracking glasses" refers to a spectacle having an attached sensor for tracking an eye. As generally used, the term "visually evoked potential device" refers to a device configured for recording of a specific part of the nervous system.

In a particularly preferred embodiment, the apparatus may further be comprising
- at least one head tracking device;
wherein the processing device is further designated for determining the visual parameter of the at least one eye of the person by using a measured value for the head movement. The head tracking device may be the camera, particularly at least one of the front camera and/or the back camera of the smartphone.

In a particularly preferred embodiment, the apparatus may further be comprising
- at least one distance measuring unit configured for measuring a distance between the screen and the at least one eye of the person,
wherein the processing device is further designated for determining the visual parameter of the at least one eye of the person by using a measured value for the distance between the screen and the at least one eye of the person. The distance measuring unit may be the camera, particularly at least one of the front camera and/or the back camera of the smartphone.

In a particularly preferred embodiment, the apparatus may further be comprising
- at least one communication unit, wherein the at least one communication unit may, preferably, be configured for at least one of:
   ∘ forwarding the tracking data to at least one external storage unit, and receiving the tracking data from the at least one external storage unit for further processing by the processing device;
   ∘ forwarding the tracking data and further data related to the at least one first visual stimulus and/or the at least one second visual stimulus to at least one external processing device, and receiving still further data related to the at least one visual parameter of the at least one eye of the person from the at least one external processing device.

As used herein, the term "external" refers to a unit with which a communication between the apparatus and the unit is performed via a network, particularly via a network system comprising a plurality of computers and/or computer networks, more particularly via the internet. As generally used, the term "storage unit" refers to at least one component and/or at least one recording media capable of retaining digital data.

In a particularly preferred embodiment, the at least one external storage unit may be comprised by at least one of a local server, a remote server, or a cloud server. As used herein, the term "local" refers to a server that is located at the location of the apparatus. As used herein, the term "remote" refers to a server that is located at a different location than the apparatus. As generally used, the term "cloud" refers to a sever at a different location, wherein a communication between the apparatus and the server is possible via the internet. In a particularly preferred embodiment, the at least one external processing device may be comprised by at least one of a local server, a remote server, or a cloud server. In a particularly preferred embodiment, the apparatus may further be configured for carrying out the method according to any one of the preceding method Embodiments.

With respect to the prior art, the method and device according to the present invention exhibits the following advantages.

Visual parameter tests for determining a visual parameter from the state of art mostly require an ophthalmologist or optometry specialist. Therefore, such test result in reduced portability and cannot be performed by a person itself. The automated test of the present invention on the other hand may be performed by the person itself, particularly by using a mobile device.

As the eye movement is measured directly using an eye tracking device, the test has the advantage that it does not require any further response of the patient. This makes testing of children or patients with disabilities easier.

The testing is time efficient, as a one-trial-only testing procedure may be performed, particularly in combination with a smooth enhancement of the visual stimulus, and particularly by considering the first threshold and the second threshold as well as psychometric procedures.

Due to the displaying the at least one first visual stimulus and the at least one second visual stimulus, particularly with at least one differing visual stimulus parameter, at the same time on the screen the method is more robust and more exact in determining the visual parameter compared to known methods wherein only one visual stimulus is being displayed.

By using at least two dynamic visual stimuli each having at least a moving spatial portion the amount of information about the at least one visual parameter included in the resulting eye movement can be increased, in particular when compared to displaying a visual stimulus designated for eliciting a fixational eye movement in combination with a visual stimulus designated for eliciting an optokinetic nystagmus. The measurement is, thus, more reliable, and may require less time without sacrificing accuracy.

By using at least one pursuit stimulus, the determination of the at least one visual parameter may be implemented using game design principles, game design thinking and/or game mechanics more easily. This makes the measurement procedure more enjoyable for the person.

In addition, the determination of the at least one visual parameter is, in general, more independent from external factors or effects on the movement of the eye, such as motivation and attention of the person.

In particular for two visual stimuli being the same, a relative measurement method can be used, which may, advantageously, cause the method to have no or only very few requirements with respect to screen calibration, distance between the at least one eye of the person and the screen, or ambient light level.

As used herein, the terms "have", "comprise" or "include" or any arbitrary grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

### Short description of the Figures

Further optional features and embodiments of the present invention are disclosed in more detail in the subsequent description of preferred embodiments, preferably in conjunction with the dependent claims. Therein, the respective optional features may be implemented in an isolated fashion as well as in any arbitrary feasible combination, as the skilled person will realize. It is emphasized here that the scope of the invention is not restricted by the preferred embodiments.

In the Figures:
- Figure 1: illustrates an aerial view of an exemplary apparatus for determining at least one visual parameter of at least one eye of a person;
- Figure 2: illustrates a schematic view of a method for determining at least one visual parameter of at least one eye of a person implemented as a computer program running on the apparatus; and
- Figure 3: illustrates a schematic view of a screen showing an exemplary embodiment of two pursuit stimuli departing from a coinciding spatial location.

### Detailed description of the embodiments

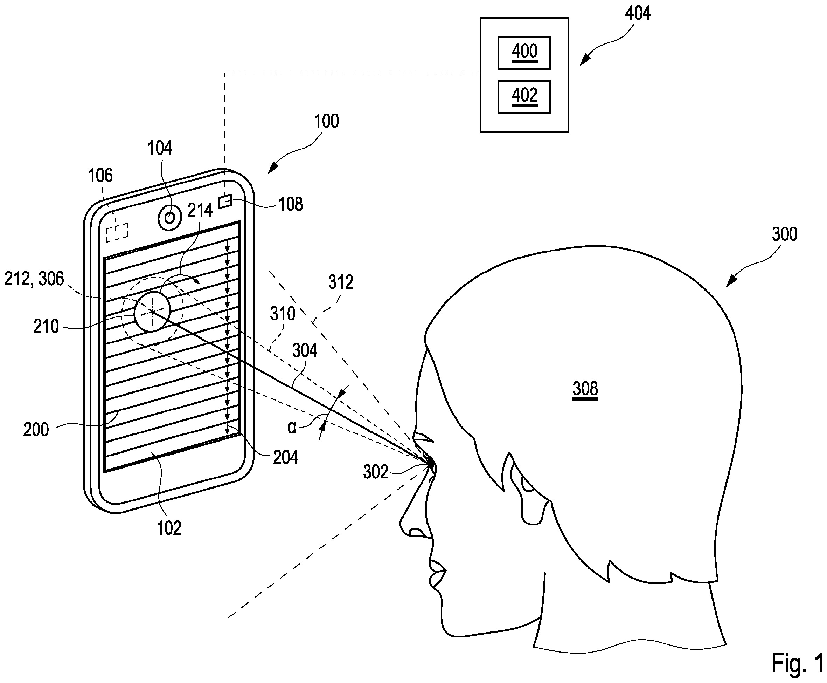

Figure 1 shows an exemplary apparatus 100 for determining a visual parameter of at least one eye 302 of a person 300. The at least one visual parameter of the at least one eye 302 of the person 300 may be selected from at least one of a refractive error or a visual performance of the at least one eye 302 of the person 300. The refractive error may be at least one of a value related to: a spherical power; a cylindrical power; a cylinder axis;; or an addition. The visual performance may be selected from at least one of: a visual acuity, particularly a near field visual acuity and/or a far field visual acuity; a contrast sensitivity; a color vision; or a visual field.

The apparatus 100 is comprising a screen 102. The screen 102 is configured for displaying to the at least one eye 302 of the person 300 a first visual stimulus 200. The first visual stimulus 200, which is used in this exemplary embodiment, is an optokinetic nystagmus stimulus. The optokinetic nystagmus stimulus is designated to elicit an optokinetic nystagmus, particularly to elicit the reflexive eye movement.

For this purpose, the optokinetic nystagmus stimulus has a structured appearance. The structured appearance is shifting, particularly translationally shifting, in one direction as indicated by the arrows. Therefore, at least one at least a portion of the first visual stimulus 200 has a first moving spatial location 204. The optokinetic nystagmus stimulus structure is, as schematically depicted in Figure 1, provided by a Gabor patch having a stripe pattern. Alternatively, the optokinetic nystagmus stimulus may be Gabor patch having a sinusoidal pattern, a noise patch having a sinusoidal pattern or a noise patch having a stripe pattern. Other patterns are also possible.

A first spatial frequency of the Gabor patch is eliciting the optokinetic nystagmus in a first direction and is a visual stimulus parameter of the optokinetic nystagmus stimulus. Additionally, a second spatial frequency may elicit the optokinetic nystagmus in a second direction and may be a further visual stimulus parameter of the optokinetic nystagmus stimulus. The first direction and the second direction may be different from each other. The first direction of the optokinetic nystagmus may be elicted and the second direction of the optokinetic nystagmus may be elicited subsequently. Additionally, a third spatial frequency eliciting the optokinetic nystagmus in a third direction may be a further visual stimulus parameter of the optokinetic nystagmus stimulus. The third direction of the optokinetic nystagmus elicited by the third spatial frequency may be different from the first direction of the optokinetic nystagmus elicited by the first spatial frequency and the second direction of the optokinetic nystagmus elicited by the second spatial frequency. Additionally, the optokinetic nystagmus stimulus may be blurred.

The optokinetic nystagmus comprises a slow phase and a quick phase, wherein in the slow phase a pursuit eye movement is elicited by the spatial frequency, and wherein in the quick phase a saccadic eye movement is a reset movement of the at least one eye 302.

The screen 102 is further configured for displaying to the at least one eye 302 of a person 300 a second visual stimulus 210. The second visual stimulus 210, which is used in this exemplary embodiment, is a pursuit stimulus. Therefore, the second visual stimulus 210 elicits a second eye movement, namely a pursuit eye movement. The pursuit eye movement may be a conscious eye movement.

The pursuit stimulus is, as schematically depicted in Figure 1, a circle. Alternatively, the pursuit stimulus may be a Gabor patch; a noise patch, particularly having a predefined spatial frequency; a ring structure, particularly a ring structure having a plurality of rings having a defined radial spatial frequency; a grid, particularly a grid comprising gabor patches of different tilts and/or spatial frequencies; a star; a letter, particularly selected from a tumbling E and/or a Landolt C.

The second visual stimulus 210 has a spatial location, particularly a spatial location of a center 212, of the pursuit stimulus that is time varying. The gaze position 306 of the person 300 is coinciding with the center 212. Therefore, a spatial movement speed is not 0, as indicated by the arrow, and at least a portion of the second visual stimulus 210 has a second moving spatial location 214. The appearance of the pursuit stimulus is maintained, and it is particularly not translationally shifting. Additionally, the pursuit stimulus may be blurred.

As shown in Figure 1, the first visual stimulus 200 and the second visual stimulus 210 are displayed at the same time on the screen 102 effecting a resulting eye movement depending on the at least one visual parameter. The first visual stimulus 200 and the second visual stimulus 210 are both perceptible from the at least one eye 302 of the person 300 in a manner that the resulting eye movement is generated by the first visual stimulus 200 and/or the second visual stimulus 210.The first visual stimulus 200 and the second visual stimulus 210 may cause the resulting eye movement depending on the at least one visual parameter of the at least one eye 302 of the person 300 to be determined.

The first visual stimulus 200 and the second visual stimulus 210 are displayed in such a manner that the first visual stimulus 200 and the second visual stimulus 210 are visible to the at least one eye 302 of the person 300 at the same time. The first visual stimulus 200 is visible to a central field of view 310 of the at least one eye 302 of the person 300. The second visual stimulus 210 is visible to the central field of view 310 of the at least one eye 302 of the person 300 and the peripheral field of view 312 of the at least one eye 302 of the person 300. A central field of view opening angle α as exemparily shown in Figure 1 is 8°.

A display area of the first visual stimulus 200 is larger than a display area of the second visual stimulus 210. The display area of the first visual stimulus 200 as schematically illustrated in Figure 1 comprises completely the display area of the second visual stimulus 210.

The apparatus 100 further comprises an eye-tracking device 104, wherein the eye-tracking device 104 is configured for generating tracking data about the resulting eye movement of the at least one eye 302 of the person 300. The eye-tracking device 104, which is used in the exemplarily embodiment of Figure 1, is a camera, particularly a front camera the smartphone. Alternatively, the eye-tracking device 104 may be a webcam; eye tracking glasses; a back camera the smartphone; or a visually evoked potential device.

The apparatus 100 further comprises a processing device 106. The processing device 106 is configured for determining the at least one visual parameter of the at least one eye 302 of the person 300 by comparing the tracking data, the first moving spatial location 204 and the second moving spatial location 214.

As further illustrated in Figure 1, the screen 102, the eye-tracking device 104 and the processing device 106 are comprised by a mobile communication device. The mobile communication device as exemplarily used here is a smartphone. Alternatively, the mobile communication device may be a tablet or a laptop. Using the smartphone or, alternatively, the tablet or laptop is, particularly, preferred for the present invention, especially owing to their widespread use and easy availability all over the world, including developing countries. Alternatively, the screen 102, the eye-tracking device 104 and the processing device 106 may be comprised by a virtual reality headset; an augmented reality system; a desktop computer; a television set; or smart glasses. The processing device 106 can be comprised by a different device than the device the screen 102 and/or the eye-tracking device 104.

The apparatus 100 may further comprise at least one head tracking device, wherein the processing device 106 is further designated for determining the visual parameter of the at least one eye 302 of the person 300 by using a measured value for the head movement. Further, the front camera may be the head tracking device.

The apparatus 100 may further comprise at least one distance measuring unit configured for measuring a distance between the screen 102 and the at least one eye 302 of the person 300, wherein the processing device 106 may further be designated for determining the visual parameter of the at least one eye 302 of the person 300 by using a measured value for the distance between the screen 102 and the at least one eye 302 of the person 300. The front camera may be the distance measuring unit.

The exemplary apparatus 100 as schematically depicted in Figure 1 further comprises a communication unit 108, wherein the communication unit 108 is configured for forwarding the tracking data to an external storage unit 400, and receiving the tracking data from the external storage unit 400 for further processing by the processing device 106. The communication unit 108 as exemparily used here is further configured for forwarding the tracking data and further data related to the first visual stimulus 200 and/or the second visual stimulus to an external processing device 402, and receiving still further data related to the at least one visual parameter of the at least one eye 302 of the person 300 from the external processing device 402.

The external storage unit 400 is comprised here by a local server 404. Alternatively, the external storage unit 400 may be comprised by a remote server, or a cloud server. The at least one external processing device 402 is also comprised by the local server 404. Alternatively, the external processing device 402 may be comprised by a remote server, or a cloud server.

A computer program comprising instructions which, when the program is executed by the apparatus 100, cause the apparatus 100 to carry out a computer-implemented method 500 for determining at least one visual parameter of at least one eye 302 of a person 300 is running on the apparatus 100.

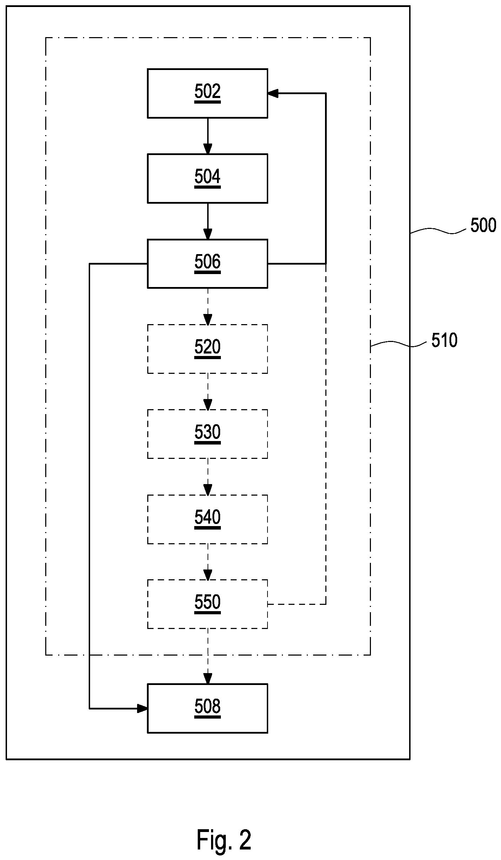

The computer-implemented method 500 is schematically illustrated in Figure 2 and comprises a first displaying step 502 according to step a) of the computer-implemented method 500. In the first displaying step 502, the first visual stimulus 200, having at least a moving portion with a first moving spatial location 204, is displayed on the screen 102 to the at least one eye 302 of the person 300.

The computer-implemented method 500 comprises further a second displaying step 504 according to step b) of the computer-implemented method 500. In the second displaying step 504, the second visual stimulus 210, having at least a moving portion with a second moving spatial location 214, is displayed on the screen 102 to the at least one eye 302 of the person 300.

The first visual stimulus 200 and the second visual stimulus 210 are displayed at the same time on the screen 102 effecting a resulting eye movement depending on the at least one visual parameter. The term "at the same time" refers to displaying the first visual stimulus at 200 a first display time and the at least one second visual stimulus at a second display time, wherein the first display time and the second display time overlap partially or fully. The computer-implemented method 500 comprises further a tracking step 506 according to step c) of the computer-implemented method 500. In the tracking step 506, tracking data about the resulting eye movement of the at least one eye 302 of the person 300 is generated by using the eye-tracking device 104.

The computer-implemented method 500 comprises further a determining step 508 according to step d) of the computer-implemented method 500. In the determining step 508, the at least one visual parameter of the at least one eye 302 of the person 300 is determined by comparing the tracking data, the first moving spatial location 204 and the second moving spatial location 214 by using the processing device 106.

The first visual stimulus 200 is defined by using at least one first visual stimulus parameter, and the second visual stimulus 210 is defined by using at least one second visual stimulus parameter. The at least one first visual stimulus parameter and the at least one second visual stimulus parameter differ from each other, by being different paramenters or having a different value for the same paramenter. The first visual stimulus parameter and the second visual stimulus parameter may further be compared for determining the at least one visual parameter.

The at least one first visual stimulus parameter or the at least one second visual stimulus parameter are selected from at least one of: a spatial frequency; a spatial frequency range; or a contrast level; of the first visual stimulus 200 or the second visual stimulus 210.

The at least one first visual stimulus parameter or the at least one second visual stimulus parameter may be varied over time, specifically in a continuous manner, particularly in a monotonous manner; or in a stepwise manner. The at least one first visual stimulus parameter and the at least one second visual stimulus parameter may be varied in succession, particularly the at least one first visual stimulus parameter may be varied when the at least one second visual stimulus parameter is mainained constant, or vice versa. Generating the tracking data may further comprise recording a time stamp at which the at least one resulting eye movement occurs for the first time or the last time.

The computer-implemented method 500 may, further, comprise a distance recording step 520 according to step e) of the computer-implemented method 500, in which at least one distance between the at least one eye 302 of the person 300 and the screen 102 displaying at least one of the first visual stimulus 200 or the second visual stimulus 210 may be recorded.

The computer-implemented method 500 may, further, comprise a line of sight 304 recording step 530 according to step f) of the computer-implemented method 500, in which at least one line of sight 304 of the at least one eye 302 of the person 300 may be recorded.

The computer-implemented method 500 may, further, comprise a gaze position recording step 540 according to step g) of the computer-implemented method 500, in which at least one gaze position 306 of the at least one eye 302 of the person 300 may be recorded.

The computer-implemented method 500 may, further, comprise a head movement recording step 550 according to step h) of the computer-implemented method 500, in which a head movement of the head 308 of the person 300 comprising the at least one eye 302 may be recorded.

The first displaying step 502 and the second displaying step 504 are performed at the same time. The tracking step 506 is performed during the first displaying step 502 and the second displaying step 504. The first displaying step 502, the second displaying step 504 and the tracking step 506 define a measurement cycle 510. The measurement cycle 510 may further comprise the distance recording step 520, the line of sight recording step 530, the gaze position recording step 540 and/or the head movement recording step 550. At least 2; at least 5; at least 10; at least 50; or at least 100 measurement cylces 510 may be performed.

An indication of the at least one visual stimulus parameter of the first visual stimulus 200 or the at least one visual stimulus parameter the second visual stimulus 210 may be requested from the person 300. The request may be a visual prompt; an auditive prompt; or a tactile prompt.

Determining the at least one visual parameter of the at least one eye 302 of the person 300 may, preferably, comprise analyzing at least one outcome. The at least one outcome may, particularly, comprise: the tracking data about the at least one resulting eye movement, particularly selected from at least one of: the at least one gaze position 306 of the at least one eye 302 of the person 300; or the at least one line of sight 304 of the at least one eye 302 of the person 300; and the first moving spatial location 204 and the second moving spatial location 214, particularly selected from at least one of: the first visual stimulus 200; the second visual stimulus; and wherein at least one outcome further comprises at least one of: the at least one visual stimulus parameter of the first visual stimulus 200; or the at least one visual stimulus parameter of the second visual stimulus. The at least one outcome may further comprise at least one of: the tracking data about the at least one head movement of the head 308 of the person 300; or the at least one distance between the at least one eye 302 of the person 300 and the screen 102 displaying at least one of the first visual stimulus 200 or the second visual stimulus.

Determining the visual parameter by analyzing the outcome may, preferably, be performed by using at least one of: an analytical method; a regression method; a statistical analysis, particularly a Multivariate statistic analysis, more particularly a Principle Component analysis; or a machine learning algorithm. The machine learning algorithm may be trained for determining of the visual parameter by providing training data, comprising tracking data about the at least one resulting eye movement; stimulus data about the first visual stimulus 200 and the second visual stimulus 210, particularly the spatial location of the first visual stimulus 200 and the spatial location of the at least one second visual stimulus; known data about the visual parameter; determining preliminary data about the visual parameter by using the tracking data and the stimulus data; and determining a deviation between the preliminary data about to the visual parameter and the known data about the visual parameter, adjusting the machine learning algorithm intended for minimizing the deviation; wherein the step of training is repeated until the deviation is below a threshold.

Analyzing the tracking data may, preferably, comprise analyzing the at least one resulting eye movement in the slow phase. Analyzing the at least one resulting eye movement in the slow phase of the optokinetic nystagmus may, particularly, comprise determining a velocity of the eye movement. Analyzing the tracking data may, especially, comprise analyzing at least one of: a latency; an acceleration; or a velocity of the at least one eye movement.

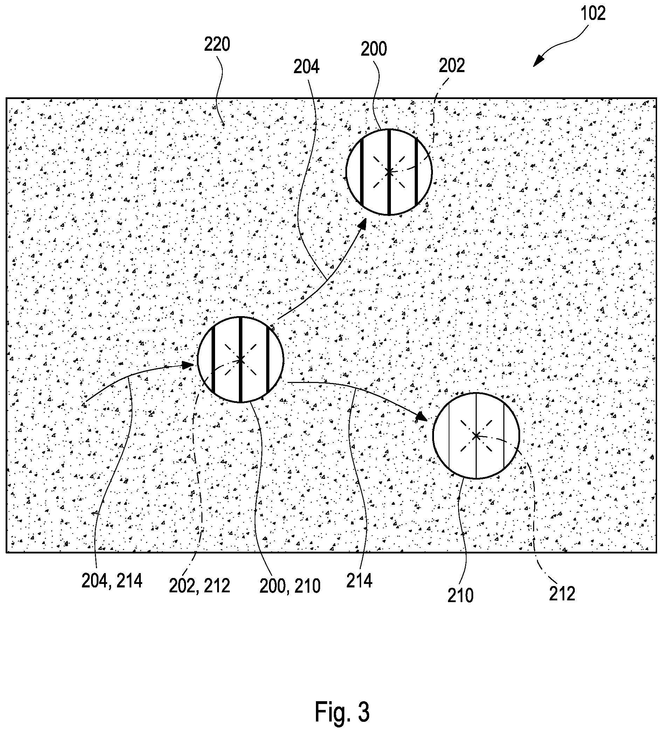

As schematically illustrated in Figure 3, the first visual stimulus 200 is a pursuit stimulus while the second visual stimulus 210 also a pursuit stimulus. The spatial location of the center 202 of the first visual stimulus 200 and the spatial location of the center 212 of the second visual stimulus 200 are coinciding at a coinciding spatial location. Further, the coinciding spatial location is moving as depicted by the arrow pointing towards the first visual stimulus 200 and the second visual stimulus 210.

In addition, a third visual stimulus 220 is displayed on the screen 102 in the exemplary embodiment of Figure 3 to the at least one eye 302 of the person 300 during the first displaying step 502 and the second displaying step 504. The third visual stimulus 220 as schematically depicted here is a noise patch. The noise patch used for this purpose here is a static noise. Alternatively, the noise patch may be a spatial frequency filtered noise; a dynamic noise; or a dynamic spatial frequency filtered noise. The noise patch may perform a translational movement.

As further illustrated in Figure 3, the first visual stimulus 200 and the second visual stimulus 210 move, starting from the coinciding spatial location, in such a manner that the spatial location, particularly of the center 202, of the first visual stimulus 200 and the spatial location, particularly of the center 212, of the second visual stimulus 210 are no longer coinciding. The movement is indicated here by the arrows poiting away from the coinciding spatial location. For this purpose, the first visual stimulus 200 and the second visual stimulus 210 move with a spatial movement direction and a spatial movement speed being different from each other.

The first visual stimulus 200 and the second visual stimulus 210 as used for this purpose here are different in at least one further visual stimulus parameter selected from at least one of: a spatial frequency; a spatial frequency range; or a contrast level.

A plurality of the first visual stimulus 200 may be displayed during the first displaying step 502 and/or a plurality of the second visual stimulus 210 may be displayed during the second displaying step 504.

Further, the first visual stimulus 200 or the second visual stimulus 210 may perform a transition from a visual stimulus designated for eliciting a given eye movement to a visual stimulus designated for eliciting a different eye movement. The first visual stimulus 200 or the second visual stimulus 200 may perform the transition from a pursuit stimulus to an optokinetic nystagmus stimulus; or vice versa.

### List of Reference Signs

- 100: apparatus for determining a visual parameter of at least one eye of a person
- 102: screen
- 104: eye-tracking device
- 106: processing device
- 108: communication unit
- 200: first visual stimulus
- 202: center
- 204: first moving spatial location
- 210: second visual stimulus
- 212: center
- 214: second moving spatial location
- 220: third visual stimulus
- 300: person
- 302: eye
- 304: line of sight
- 306: gaze position
- 308: head
- 310: central field of view
- 312: peripheral field of view
- 400: external storage unit
- 402: external processing device
- 404: local server
- 500: computer-implemented method
- 502: first displaying step
- 504: second displaying step
- 506: tracking step
- 508: determining step
- 510: measurement cycle
- 520: distance recording step
- 530: line of sight recording step
- 540: gaze position recording step
- 550: head movement recording step
- α: angle

## Claims

1. A computer-implemented method (500) for determining at least one visual parameter of at least one eye (302) of a person (300), the method (500) comprising the following steps:
a) displaying (502) on a screen (102) to at least one eye (302) of a person (300) at least one first visual stimulus (200), wherein at least a portion of the at least one first visual stimulus (200) has a first moving spatial location (204); and
b) displaying (504) on the screen (102) to the at least one eye (302) of the person (300) at least one second visual stimulus (210), wherein at least a portion of the at least one second visual stimulus (210) has a second moving spatial location (214);
wherein the at least one first visual stimulus (200) and the at least one second visual stimulus (210) are displayed at the same time on the screen (102) effecting a resulting eye movement depending on the at least one visual parameter;
c) generating tracking data (506) about the resulting eye movement of the at least one eye (302) of the person (300) by using at least one eye-tracking device (104); and
d) determining (508) at least one visual parameter of the at least one eye (302) of the person (300) by comparing the tracking data, the first moving spatial location (204) and the second moving spatial location (214) by using at least one processing device (106);
wherein the at least one first visual stimulus (200) is a pursuit stimulus and the at least one second visual stimulus (210) is a pursuit stimulus,
wherein the spatial location of the at least one first visual stimulus (200) and the spatial location of the at least one second visual stimulus (210) are coinciding at a coinciding spatial location, wherein the at least one first visual stimulus (200) and the at least one second visual stimulus (210) move, starting from the coinciding spatial location, in such a manner that the spatial location of the at least one first visual stimulus (200) and the spatial location of the at least one second visual stimulus (210) are no longer coinciding,
**characterized in that**
the coinciding spatial location is a matching spatial location, wherein at this matching spatial location at least a portion of the at least one first visual stimulus (200) covers up at least a portion of the at least one second visual stimulus (210) or vice versa in such a manner that the covered portion of the at least one second visual stimulus (210) or the covered portion of the at least one first visual stimulus (200) is not perceptible by the at least one eye of the person,
wherein the at least one first visual stimulus and the at least one second visual stimulus move, starting from the coinciding spatial location, with at least one of a spatial movement direction or a spatial movement speed being different from each other, thereby, the at least one first visual stimulus and the at least one second visual stimulus move away from the coinciding position and/or away from one another,
wherein the at least one first visual stimulus and the at least one second visual stimulus are different in at least one further visual stimulus parameter selected from at least one of: a spatial frequency, a spatial frequency range, or a contrast level.

2. The method (500) according to the preceding claim, wherein the at least one visual parameter of the at least one eye (302) of the person (300) is selected from at least one of a refractive error or a visual performance of the at least one eye (302) of the person (300).

3. The method (500) according to the preceding claim, wherein the refractive error of the at least one eye (302) of the person (300) is at least one of a value related to:
- a spherical power;
- a cylinder;
- a cylinder axis; or
- an addition.

4. The method (500) according to any of the two preceding claims, wherein the visual performance is selected from at least one of
- a visual acuity, particularly selected from at least one of:
∘ a near field visual acuity; or
∘ a far field visual acuity;
- a contrast sensitivity;
- a color vision; or
- a visual field.

5. The method (500) according to any one of the preceding claims, wherein an indication of at least one of:
- the at least one visual stimulus parameter of the at least one first visual stimulus (200); or
- the at least one visual stimulus parameter of the at least one second visual stimulus (210);
is requested from the person (300).

6. The method (500) according to any one of the preceding claims, wherein the at least one first visual stimulus (200) or the at least one second visual stimulus (210) performs the transition from a pursuit stimulus to an optokinetic nystagmus stimulus; or vice versa, wherein at least one of: the at least one first visual stimulus (200); or the at least one second visual stimulus (210) remains the pursuit stimulus.

7. The method (500) according to any one of the preceding claims, wherein at least one outcome comprises:
- the tracking data about the at least one resulting eye movement, particularly selected from at least one of:
∘ the at least one gaze position (306) of the at least one eye (302) of the person (300); or
∘ the at least one line of sight (304) of the at least one eye (302) of the person (300); and
- the first moving spatial location (204) and the second moving spatial location (214), particularly selected from at least one of:
∘ the at least one first visual stimulus (200);
∘ the at least one second visual stimulus (210); and
wherein at least one outcome further comprises at least one of:
- the at least one visual stimulus parameter of the at least one first visual stimulus (200); or
- the at least one visual stimulus parameter of the at least one second visual stimulus (210).

8. The method (500) according to any one of the preceding claims, wherein determining the visual parameter by analyzing the outcome is performed by using at least one of:
- an analytical method;
- a regression method;
- a statistical analysis, particularly a Multivariate statistic analysis, more particularly a Principle Component analysis; or
- a machine learning algorithm.

9. A computer program comprising instructions which, when the program is executed by a computer, cause the computer to carry out a computer-implemented method (500) for determining at least one visual parameter of at least one eye (302) of a person (300), the method (500) comprising the following steps:
a) displaying (502) on a screen (102) to at least one eye (302) of a person (300) at least one first visual stimulus (200), wherein at least a portion of the at least one first visual stimulus (200) has a first moving spatial location (204); and
b) displaying (504) on the screen (102) to the at least one eye (302) of the person (300) at least one second visual stimulus (210), wherein at least a portion of the at least one second visual stimulus (210) has a second moving spatial location (214);
wherein the at least one first visual stimulus (200) and the at least one second visual stimulus (210) are displayed at the same time on the screen (102) effecting a resulting eye movement depending on the at least one visual parameter;
c) generating (506) tracking data about the resulting eye movement of the at least one eye (302) of the person (300) by using at least one eye-tracking device (104); and
d) determining (508) at least one visual parameter of the at least one eye (302) of the person (300) by comparing the tracking data, the first moving spatial location (204) and the second moving spatial location (214) by using at least one processing device (106);
wherein the at least one first visual stimulus (200) is a pursuit stimulus and the at least one second visual stimulus (210) is a pursuit stimulus, wherein the spatial location of the at least one first visual stimulus (200) and the spatial location of the at least one second visual stimulus (210) are coinciding at a coinciding spatial location, wherein the at least one first visual stimulus (200) and the at least one second visual stimulus (210) move, starting from the coinciding spatial location, in such a manner that the spatial location of the at least one first visual stimulus (200) and the spatial location of the at least one second visual stimulus (210) are no longer coinciding,
**characterized in that**
the coinciding spatial location is a matching spatial location, wherein at this matching spatial location at least a portion of the at least one first visual stimulus (200) covers up at least a portion of the at least one second visual stimulus (210) or vice versa in such a manner that the covered portion of the at least one second visual stimulus (210) or the covered portion of the at least one first visual stimulus (200) is not perceptible by the at least one eye of the person,
wherein the at least one first visual stimulus and the at least one second visual stimulus move, starting from the coinciding spatial location, with at least one of a spatial movement direction or a spatial movement speed being different from each other, thereby, the at least one first visual stimulus and the at least one second visual stimulus move away from the coinciding position and/or away from one another,
wherein the at least one first visual stimulus and the at least one second visual stimulus are different in at least one further visual stimulus parameter selected from at least one of: a spatial frequency, a spatial frequency range, or a contrast level.

10. An apparatus (100) for determining a visual parameter of at least one eye (302) of a person (300), the apparatus (100) comprising:
- at least one screen (102), wherein the at least one screen (102) is configured for displaying to the at least one eye (302) of a person (300)
∘ at least one first visual stimulus (200), wherein at least a portion of the at least one first visual stimulus (200) has a first moving spatial location (204); and
∘ at least one second visual stimulus (210), wherein at least a portion of the at least one second visual stimulus (210) has a second moving spatial location (214);
∘ wherein the at least one first visual stimulus (200) and the at least one second visual stimulus (210) are displayed at the same time on the screen (102) effecting a resulting eye movement depending on the at least one visual parameter;
- at least one eye-tracking device (104), wherein the at least one eye-tracking device (104) is configured for generating tracking data about the resulting eye movement of the at least one eye (302) of the person (300); and
- at least one processing device (106), wherein the at least one processing device (106) is configured for determining at least one visual parameter of the at least one eye (302) of the person (300) by comparing the tracking data, the first moving spatial location (204) and the second moving spatial location (214),
wherein the at least one first visual stimulus (200) is a pursuit stimulus and the at least one second visual stimulus (210) is a pursuit stimulus, wherein the spatial location of the at least one first visual stimulus (200) and the spatial location of the at least one second visual stimulus (210) are coinciding at a coinciding spatial location, wherein the at least one first visual stimulus (200) and the at least one second visual stimulus (210) move, starting from the coinciding spatial location, in such a manner that the spatial location of the at least one first visual stimulus (200) and the spatial location of the at least one second visual stimulus (210) are no longer coinciding,
**characterized in that**
the coinciding spatial location is a matching spatial location, wherein at this matching spatial location at least a portion of the at least one first visual stimulus (200) covers up at least a portion of the at least one second visual stimulus (210) or vice versa in such a manner that the covered portion of the at least one second visual stimulus (210) or the covered portion of the at least one first visual stimulus (200) is not perceptible by the at least one eye of the person,
wherein the at least one first visual stimulus and the at least one second visual stimulus move, starting from the coinciding spatial location, with at least one of a spatial movement direction or a spatial movement speed being different from each other, thereby, the at least one first visual stimulus and the at least one second visual stimulus move away from the coinciding position and/or away from one another,
wherein the at least one first visual stimulus and the at least one second visual stimulus are different in at least one further visual stimulus parameter selected from at least one of: a spatial frequency, a spatial frequency range, or a contrast level.

## Patentansprüche

1. Computerimplementiertes Verfahren (500) zum Bestimmen mindestens eines visuellen Parameters mindestens eines Auges (302) einer Person (300), wobei das Verfahren (500) die folgenden Schritte umfasst:
a) Anzeigen (502) auf einem Bildschirm (102) für mindestens ein Auge (302) einer Person (300) mindestens eines ersten visuellen Reizes (200), wobei mindestens ein Teil des mindestens einen ersten visuellen Reizes (200) einen ersten sich bewegenden räumlichen Ort (204) aufweist; und
b) Anzeigen (504) auf dem Bildschirm (102) für mindestens ein Auge (302) der Person (300) mindestens eines zweiten visuellen Reizes (210), wobei mindestens ein Teil des mindestens einen zweiten visuellen Reizes (210) einen zweiten sich bewegenden räumlichen Ort (214) aufweist; wobei der mindestens eine erste visuelle Reiz (200) und der mindestens eine zweite visuelle Reiz (210) gleichzeitig auf dem Bildschirm (102) angezeigt werden, was eine resultierende Augenbewegung in Abhängigkeit von dem mindestens einen visuellen Parameter bewirkt;
c) Erzeugen von Trackingdaten (506) über die resultierende Augenbewegung des mindestens einen Auges (302) der Person (300) unter Verwendung mindestens einer Augentrackingvorrichtung (104); und
d) Bestimmen (508) mindestens eines visuellen Parameters des mindestens einen Auges (302) der Person (300) durch Vergleichen der Trackingdaten, des ersten sich bewegenden räumlichen Orts (204) und des zweiten sich bewegenden räumlichen Orts (214) unter Verwendung mindestens einer Verarbeitungsvorrichtung (106);
wobei der mindestens eine erste visuelle Reiz (200) ein Verfolgungsreiz ist und der mindestens eine zweite visuelle Reiz (210) ein Verfolgungsreiz ist,
wobei der räumliche Ort des mindestens einen ersten visuellen Reizes (200) und der räumliche Ort des mindestens einen zweiten visuellen Reizes (210) an einem zusammenfallenden räumlichen Ort zusammenfallen, wobei sich der mindestens eine erste visuelle Reiz (200) und der mindestens eine zweite visuelle Reiz (210) ausgehend von dem zusammenfallenden räumlichen Ort bewegen, auf eine solche Weise, dass der räumliche Ort des mindestens einen ersten visuellen Reizes (200) und der räumliche Ort des mindestens einen zweiten visuellen Reizes (210) nicht mehr zusammenfallen,
**dadurch gekennzeichnet, dass**
der zusammenfallende räumliche Ort ein übereinstimmender räumlicher Ort ist, wobei an dieser übereinstimmenden räumlichen Position mindestens ein Teil des mindestens einen ersten visuellen Reizes (200) mindestens einen Teil des mindestens einen zweiten visuellen Reizes (210) abdeckt oder umgekehrt, derart, dass der abgedeckte Teil des mindestens einen zweiten visuellen Reizes (210) oder der abgedeckte Teil des mindestens einen ersten visuellen Reizes (200) durch das mindestens eine Auge der Person nicht wahrnehmbar ist,
wobei sich der mindestens eine erste visuelle Reiz und der mindestens eine zweite visuelle Reiz ausgehend von dem zusammenfallenden räumlichen Ort bewegen, wobei sich eine räumliche Bewegungsrichtung und/oder eine räumliche Bewegungsgeschwindigkeit voneinander unterscheiden, wodurch sich der mindestens eine erste visuelle Reiz und der mindestens eine zweite visuelle Reiz von der zusammenfallenden Position wegbewegen und/oder voneinander wegbewegen,
wobei sich der mindestens eine erste visuelle Reiz und der mindestens eine zweite visuelle Reiz in mindestens einem weiteren visuellen Reizparameter unterscheiden, der aus mindestens einem von Folgendem ausgewählt ist: einer Raumfrequenz, einem Raumfrequenzbereich oder einem Kontrastniveau.

2. Verfahren (500) nach dem vorhergehenden Anspruch, wobei der mindestens eine visuelle Parameter des mindestens einen Auges (302) der Person (300) aus mindestens einem von einem Brechungsfehler oder einer visuellen Leistungsfähigkeit des mindestens einen Auges (302) der Person (300) ausgewählt ist.

3. Verfahren (500) nach dem vorhergehenden Anspruch, wobei der Brechungsfehler des mindestens einen Auges (302) der Person (300) mindestens einer von einem Wert ist, der sich auf Folgendes bezieht:
- eine sphärische Kraft;
- einen Zylinder;
- eine Zylinderachse; oder
- eine Ergänzung.

4. Verfahren (500) nach einem der zwei vorhergehenden Ansprüche, wobei die visuelle Leistung aus mindestens einem der Folgenden ausgewählt wird
- einer Sehschärfe, insbesondere ausgewählt aus mindestens einem der Folgenden:
∘ einer Nahfeld-Sehschärfe; oder
∘ einer Fernfeld-Sehschärfe;
- einer Kontrastempfindlichkeit;
- einem Farbbild; oder
- einem Sichtfeld.

5. Verfahren (500) nach einem der vorhergehenden Ansprüche, wobei eine Angabe von mindestens einem der Folgenden:
- dem mindestens einen visuellen Reizparameter des mindestens einen ersten visuellen Reizes (200); oder
- dem mindestens einen visuellen Reizparameter des mindestens einen zweiten visuellen Reizes (210);
von der Person (300) angefordert wird.

6. Verfahren (500) nach einem der vorhergehenden Ansprüche, wobei der mindestens eine erste visuelle Reiz (200) oder der mindestens eine zweite visuelle Reiz (210) den Übergang von einem Verfolgungsreiz zu einem optokinetischen Nystagmusreiz durchführt; oder umgekehrt, wobei mindestens einer der Folgenden: der mindestens eine erste visuelle Reiz (200); oder der mindestens eine zweite visuelle Reiz (210) der Verfolgungsreiz bleibt.

7. Verfahren (500) nach einem der vorhergehenden Ansprüche, wobei mindestens ein Ergebnis Folgendes umfasst:
- die Trackingdaten über die mindestens eine resultierende Augenbewegung, insbesondere ausgewählt aus mindestens einem der Folgenden:
o der mindestens einen Blickposition (306) des mindestens einen Auges (302) der Person (300); oder
o der mindestens einen Sichtlinie (304) des mindestens einen Auges (302) der Person (300); und
- dem ersten sich bewegenden räumlichen Ort (204) und dem zweiten sich bewegenden räumlichen Ort (214), insbesondere ausgewählt aus mindestens einem von:
∘ dem mindestens einen ersten visuellen Reiz (200);
∘ dem mindestens einen zweiten visuellen Reiz (210); und
wobei mindestens ein Ergebnis ferner mindestens eines der Folgenden umfasst:
- dem mindestens einen visuellen Reizparameter des mindestens einen ersten visuellen Reizes (200); oder
- dem mindestens einen visuellen Reizparameter des mindestens einen zweiten visuellen Reizes (210).

8. Verfahren (500) nach einem der vorhergehenden Ansprüche, wobei das Bestimmen des visuellen Parameters durch Analysieren des Ergebnisses unter Verwendung von mindestens einem der Folgendem durchgeführt wird:
- eines Analyseverfahrens;
- eines Regressionsverfahrens;
- einer statistischen Analyse, insbesondere einer multivariaten statistischen Analyse, insbesondere einer prinzipiellen Komponentenanalyse; oder
- eines Algorithmus für maschinelles Lernen.

9. Computerprogramm, das Anweisungen umfasst, die, wenn das Programm durch einen Computer ausgeführt wird, den Computer veranlasst, ein computerimplementiertes Verfahren (500) zum Bestimmen mindestens eines visuellen Parameters mindestens eines Auges (302) einer Person (300) durchzuführen, wobei das Verfahren (500) die folgenden Schritte umfasst:
a) Anzeigen (502) auf einem Bildschirm (102) für mindestens ein Auge (302) einer Person (300) mindestens eines ersten visuellen Reizes (200), wobei mindestens ein Teil des mindestens einen ersten visuellen Reizes (200) einen ersten sich bewegenden räumlichen Ort (204) aufweist; und
b) Anzeigen (504) auf dem Bildschirm (102) für mindestens ein Auge (302) der Person (300) mindestens eines zweiten visuellen Reizes (210), wobei mindestens ein Teil des mindestens einen zweiten visuellen Reizes (210) einen zweiten sich bewegenden räumlichen Ort (214) aufweist; wobei der mindestens eine erste visuelle Reiz (200) und der mindestens eine zweite visuelle Reiz (210) gleichzeitig auf dem Bildschirm (102) angezeigt werden, was eine resultierende Augenbewegung in Abhängigkeit von dem mindestens einen visuellen Parameter bewirkt;
c) Erzeugen (506) von Trackingdaten über die resultierende Augenbewegung des mindestens einen Auges (302) der Person (300) unter Verwendung mindestens einer Augentrackingvorrichtung (104); und
d) Bestimmen (508) mindestens eines visuellen Parameters des mindestens einen Auges (302) der Person (300) durch Vergleichen der Trackingdaten, des ersten sich bewegenden räumlichen Orts (204) und des zweiten sich bewegenden räumlichen Orts (214) unter Verwendung mindestens einer Verarbeitungsvorrichtung (106);
wobei der mindestens eine erste visuelle Reiz (200) ein Verfolgungsreiz ist und der mindestens eine zweite visuelle Reiz (210) ein Verfolgungsreiz ist, wobei der räumliche Ort des mindestens einen ersten visuellen Reizes (200) und der räumliche Ort des mindestens einen zweiten visuellen Reizes (210) an einem zusammenfallenden räumlichen Ort zusammenfallen, wobei sich der mindestens eine erste visuelle Reiz (200) und der mindestens eine zweite visuelle Reiz (210) ausgehend von dem zusammenfallenden räumlichen Ort auf eine solche Weise bewegen, dass der räumliche Ort des mindestens einen ersten visuellen Reizes (200) und der räumliche Ort des mindestens einen zweiten visuellen Reizes (210) nicht mehr zusammenfallen,
**dadurch gekennzeichnet, dass**
der zusammenfallende räumliche Ort ein übereinstimmender räumlicher Ort ist, wobei an dieser übereinstimmenden räumlichen Position mindestens ein Teil des mindestens einen ersten visuellen Reizes (200) mindestens einen Teil des mindestens einen zweiten visuellen Reizes (210) abdeckt oder umgekehrt, derart, dass der abgedeckte Teil des mindestens einen zweiten visuellen Reizes (210) oder der abgedeckte Teil des mindestens einen ersten visuellen Reizes (200) durch das mindestens eine Auge der Person nicht wahrnehmbar ist,
wobei sich der mindestens eine erste visuelle Reiz und der mindestens eine zweite visuelle Reiz ausgehend von dem zusammenfallenden räumlichen Ort bewegen, wobei sich eine räumliche Bewegungsrichtung und/oder eine räumliche Bewegungsgeschwindigkeit voneinander unterscheiden, wodurch sich der mindestens eine erste visuelle Reiz und der mindestens eine zweite visuelle Reiz von der zusammenfallenden Position wegbewegen und/oder voneinander wegbewegen,
wobei sich der mindestens eine erste visuelle Reiz und der mindestens eine zweite visuelle Reiz in mindestens einem weiteren visuellen Reizparameter unterscheiden, der aus mindestens einem von Folgendem ausgewählt ist:
einer Raumfrequenz, einem Raumfrequenzbereich oder einem Kontrastniveau.

10. Einrichtung (100) zum Bestimmen eines visuellen Parameters mindestens eines Auges (302) einer Person (300), wobei die Einrichtung (100) Folgendes umfasst:
- mindestens einen Bildschirm (102), wobei der mindestens eine Bildschirm (102) ausgelegt ist zum Anzeigen für das mindestens eine Auge (302) einer Person (300)
o mindestens eines ersten visuellen Reizes (200), wobei mindestens ein Teil des mindestens einen ersten visuellen Reizes (200) einen ersten sich bewegenden räumlichen Ort (204) aufweist; und
o mindestens eines zweiten visuellen Reizes (210), wobei mindestens ein Teil des mindestens einen zweiten visuellen Reizes (210) einen zweiten sich bewegenden räumlichen Ort (214) aufweist;
o wobei der mindestens eine erste visuelle Reiz (200) und der mindestens eine zweite visuelle Reiz (210) gleichzeitig auf dem Bildschirm (102) angezeigt werden, was eine resultierende Augenbewegung in Abhängigkeit von dem mindestens einen visuellen Parameter bewirkt;
- mindestens eine Augentrackingvorrichtung (104), wobei die mindestens eine Augentrackingvorrichtung (104) zum Erzeugen von Trackingdaten über die resultierende Augenbewegung des mindestens einen Auges (302) der Person (300) konfiguriert ist; und
- mindestens eine Verarbeitungsvorrichtung (106), wobei die mindestens eine Verarbeitungsvorrichtung (106) zum Bestimmen mindestens eines visuellen Parameters des mindestens einen Auges (302) der Person (300) durch Vergleichen der Trackingdaten, des ersten sich bewegenden räumlichen Orts (204) und des zweiten sich bewegenden räumlichen Orts (214) konfiguriert ist,
wobei der mindestens eine erste visuelle Reiz (200) ein Verfolgungsreiz ist und der mindestens eine zweite visuelle Reiz (210) ein Verfolgungsreiz ist, wobei der räumliche Ort des mindestens einen ersten visuellen Reizes (200) und der räumliche Ort des mindestens einen zweiten visuellen Reizes (210) an einem zusammenfallenden räumlichen Ort zusammenfallen, wobei sich der mindestens eine erste visuelle Reiz (200) und der mindestens eine zweite visuelle Reiz (210) ausgehend von dem zusammenfallenden räumlichen Ort auf eine solche Weise bewegen, dass der räumliche Ort des mindestens einen ersten visuellen Reizes (200) und der räumliche Ort des mindestens einen zweiten visuellen Reizes (210) nicht mehr zusammenfallen,
**dadurch gekennzeichnet, dass**
der zusammenfallende räumliche Ort ein übereinstimmender räumlicher Ort ist, wobei an dieser übereinstimmenden räumlichen Position mindestens ein Teil des mindestens einen ersten visuellen Reizes (200) mindestens einen Teil des mindestens einen zweiten visuellen Reizes (210) abdeckt oder umgekehrt, derart, dass der abgedeckte Teil des mindestens einen zweiten visuellen Reizes (210) oder der abgedeckte Teil des mindestens einen ersten visuellen Reizes (200) durch das mindestens eine Auge der Person nicht wahrnehmbar ist,
wobei sich der mindestens eine erste visuelle Reiz und der mindestens eine zweite visuelle Reiz ausgehend von dem zusammenfallenden räumlichen Ort bewegen, wobei sich eine räumliche Bewegungsrichtung und/oder eine räumliche Bewegungsgeschwindigkeit voneinander unterscheiden, wodurch sich der mindestens eine erste visuelle Reiz und der mindestens eine zweite visuelle Reiz von der zusammenfallenden Position wegbewegen und/oder voneinander wegbewegen,
wobei sich der mindestens eine erste visuelle Reiz und der mindestens eine zweite visuelle Reiz in mindestens einem weiteren visuellen Reizparameter unterscheiden, der aus mindestens einem von Folgendem ausgewählt ist: einer Raumfrequenz, einem Raumfrequenzbereich oder einem Kontrastniveau.

## Revendications

1. Procédé (500) mis en œuvre par ordinateur pour déterminer au moins un paramètre visuel d'au moins un œil (302) d'une personne (300), le procédé (500) comprenant les étapes suivantes :
a) afficher (502) sur un écran (102) pour au moins un œil (302) d'une personne (300) au moins un premier stimulus visuel (200), où au moins une partie de l'au moins un premier stimulus visuel (200) a une première position spatiale mobile (204) ; et
b) afficher (504) sur l'écran (102) pour l'au moins un œil (302) de la personne (300) au moins un deuxième stimulus visuel (210), où au moins une partie de l'au moins un deuxième stimulus visuel (210) a une deuxième position spatiale mobile (214) ;
dans lequel l'au moins un premier stimulus visuel (200) et l'au moins un deuxième stimulus visuel (210) sont affichés en même temps sur l'écran (102), ce qui entraîne un mouvement oculaire résultant en fonction de l'au moins un paramètre visuel ;
c) générer des données de suivi (506) sur le mouvement oculaire résultant de l'au moins un œil (302) de la personne (300) en utilisant au moins un dispositif de suivi oculaire (104) ; et
d) déterminer (508) au moins un paramètre visuel de l'au moins un œil (302) de la personne (300) en comparant les données de suivi, la première position spatiale mobile (204) et la deuxième position spatiale mobile (214) au moyen d'au moins un dispositif de traitement (106) ;
dans lequel l'au moins un premier stimulus visuel (200) est un stimulus de poursuite et l'au moins un deuxième stimulus visuel (210) est un stimulus de poursuite, dans lequel la position spatiale de l'au moins un premier stimulus visuel (200) et la position spatiale de l'au moins un deuxième stimulus visuel (210) coïncident à une position spatiale coïncidente, où l'au moins un premier stimulus visuel (200) et l'au moins un deuxième stimulus visuel (210) se déplacent, à partir de la position spatiale coïncidente, de manière à ce que la position spatiale de l'au moins un premier stimulus visuel (200) et la position spatiale de l'au moins un deuxième stimulus visuel (210) ne coïncident plus,
**caractérisé en ce que**
la position spatiale coïncidente est une position spatiale correspondant, où, à cette position spatiale correspondant, au moins une partie de l'au moins un premier stimulus visuel (200) recouvre au moins une partie de l'au moins un deuxième stimulus visuel (210), ou vice versa, de telle sorte que la partie recouverte de l'au moins un deuxième stimulus visuel (210) ou la partie recouverte de l'au moins un premier stimulus visuel (200) ne soit pas perceptible par l'au moins un œil de la personne,
dans lequel l'au moins un premier stimulus visuel et l'au moins un deuxième stimulus visuel se déplacent, à partir de la position spatiale coïncidente, avec au moins une direction de mouvement spatial ou une vitesse de mouvement spatial étant différente l'une de l'autre, de sorte que l'au moins un premier stimulus visuel et l'au moins un deuxième stimulus visuel s'éloignent de la position coïncidente et/ou s'éloignent l'un de l'autre, dans lequel l'au moins un premier stimulus visuel et l'au moins un deuxième stimulus visuel diffèrent par au moins un autre paramètre de stimulus visuel choisi parmi au moins l'un des paramètres suivants : une fréquence spatiale, une gamme de fréquences spatiales ou un niveau de contraste.

2. Procédé (500) selon la revendication précédente, dans lequel l'au moins un paramètre visuel de l'au moins un œil (302) de la personne (300) est choisi parmi au moins l'un des suivants : une erreur de réfraction ou une performance visuelle de l'au moins un œil (302) de la personne (300).

3. Procédé (500) selon la revendication précédente, dans lequel l'erreur de réfraction de l'au moins un œil (302) de la personne (300) est au moins une valeur liée à :
- une puissance sphérique ;
- un cylindre ;
- un axe de cylindre ; ou
- une addition.

4. Procédé (500) selon l'une quelconque des deux revendications précédentes, dans lequel la performance visuelle est sélectionnée parmi au moins l'un des éléments suivants :
- une acuité visuelle, notamment sélectionnée parmi au moins l'une des suivantes :
o une acuité visuelle de près ; ou
o une acuité visuelle de loin ;
- une sensibilité aux contrastes ;
- une vision des couleurs ; ou
- un champ visuel.

5. Procédé (500) selon l'une quelconque des revendications précédentes, dans lequel une indication d'au moins un paramètre parmi :
- l'au moins un paramètre de stimulus visuel de l'au moins un premier stimulus visuel (200) ; ou
- l'au moins un paramètre de stimulus visuel de l'au moins un deuxième stimulus visuel (210) ;
est demandé à la personne (300).

6. Procédé (500) selon l'une quelconque des revendications précédentes, dans lequel l'au moins un premier stimulus visuel (200) ou l'au moins un deuxième stimulus visuel (210) effectue la transition d'un stimulus de poursuite à un stimulus de nystagmus optocinétique, ou inversement, où au moins l'au moins un premier stimulus visuel (200) et/ou l'au moins un deuxième stimulus visuel (210) restent des stimuli de poursuite.

7. Procédé (500) selon l'une quelconque des revendications précédentes, dans lequel au moins un résultat comprend :
- les données de suivi concernant l'au moins un mouvement oculaire résultant, en particulier les données sélectionnées parmi au moins l'une des suivantes :
o l'au moins une position du regard (306) de l'au moins un œil (302) de la personne (300) ; ou
o l'au moins une ligne de visée (304) de l'au moins un œil (302) de la personne (300) ; et
- la première position spatiale mobile (204) et la deuxième position spatiale mobile (214), sélectionnée, en particulier, parmi au moins l'un des stimuli suivants :
o l'au moins un premier stimulus visuel (200) ;
o l'au moins un deuxième stimulus visuel (210) ; et
où au moins un résultat comprend en outre au moins l'un des paramètres suivants :
- l'au moins un paramètre de stimulus visuel de l'au moins un premier stimulus visuel (200) ; ou
- l'au moins un paramètre de stimulus visuel de l'au moins un deuxième stimulus visuel (210).

8. Procédé (500) selon l'une quelconque des revendications précédentes, dans lequel la détermination du paramètre visuel par analyse du résultat est réalisée en utilisant au moins l'un parmi :
- un procédé d'analyse ;
- un procédé de régression ;
- une analyse statistique, en particulier une analyse statistique multivariée, plus particulièrement une analyse en composantes principales ; ou
- un algorithme d'apprentissage automatique.

9. Programme d'ordinateur comprenant des instructions qui, lorsque le programme est exécuté par un ordinateur, amènent l'ordinateur à mettre en œuvre un procédé (500) implémenté par ordinateur pour déterminer au moins un paramètre visuel d'au moins un œil (302) d'une personne (300), le procédé (500) comprenant les étapes suivantes :
a) afficher (502) sur un écran (102) pour au moins un œil (302) d'une personne (300) au moins un premier stimulus visuel (200), où au moins une partie de l'au moins un premier stimulus visuel (200) a une première position spatiale mobile (204) ; et
b) afficher (504) sur l'écran (102) pour l'au moins un œil (302) de la personne (300) au moins un deuxième stimulus visuel (210), où au moins une partie de l'au moins un deuxième stimulus visuel (210) a une deuxième position spatiale mobile (214) ;
dans lequel l'au moins un premier stimulus visuel (200) et l'au moins un deuxième stimulus visuel (210) sont affichés en même temps sur l'écran (102), ce qui entraîne un mouvement oculaire résultant en fonction de l'au moins un paramètre visuel ;
c) générer (506) des données de suivi sur le mouvement oculaire résultant de l'au moins un œil (302) de la personne (300) en utilisant au moins un dispositif de suivi oculaire (104) ; et
d) déterminer (508) au moins un paramètre visuel de l'au moins un œil (302) de la personne (300) en comparant les données de suivi, la première position spatiale mobile (204) et la deuxième position spatiale mobile (214) au moyen d'au moins un dispositif de traitement (106) ;
dans lequel l'au moins un premier stimulus visuel (200) est un stimulus de poursuite et l'au moins un deuxième stimulus visuel (210) est un stimulus de poursuite, où la position spatiale de l'au moins un premier stimulus visuel (200) et la position spatiale de l'au moins un deuxième stimulus visuel (210) coïncident à une position spatiale coïncidente, où l'au moins un premier stimulus visuel (200) et l'au moins un deuxième stimulus visuel (210) se déplacent, à partir de la position spatiale coïncidente, de manière à ce que la position spatiale de l'au moins un premier stimulus visuel (200) et la position spatiale de l'au moins un deuxième stimulus visuel (210) ne coïncident plus,
**caractérisé en ce que**
la position spatiale coïncidente est une position spatiale correspondant, où, à cette position spatiale correspondant, au moins une partie de l'au moins un premier stimulus visuel (200) recouvre au moins une partie de l'au moins un deuxième stimulus visuel (210), ou vice versa, de telle sorte que la partie recouverte de l'au moins un deuxième stimulus visuel (210) ou la partie recouverte de l'au moins un premier stimulus visuel (200) ne soit pas perceptible par l'au moins un œil de la personne,
dans lequel l'au moins un premier stimulus visuel et l'au moins un deuxième stimulus visuel se déplacent, à partir de la position spatiale coïncidente, avec au moins une direction de mouvement spatial ou une vitesse de mouvement spatial étant différente l'une de l'autre, de sorte que l'au moins un premier stimulus visuel et l'au moins un deuxième stimulus visuel s'éloignent de la position coïncidente et/ou s'éloignent l'un de l'autre, dans lequel l'au moins un premier stimulus visuel et l'au moins un deuxième stimulus visuel diffèrent par au moins un autre paramètre de stimulus visuel choisi parmi au moins l'un des paramètres suivants : une fréquence spatiale, une gamme de fréquences spatiales ou un niveau de contraste.

10. Appareil (100) permettant de déterminer un paramètre visuel d'au moins un œil (302) d'une personne (300), l'appareil (100) comprenant :
- au moins un écran (102), où l'au moins un écran (102) est configuré pour afficher pour l'au moins un œil (302) d'une personne (300)
o au moins un premier stimulus visuel (200), où au moins une partie de l'au moins un premier stimulus visuel (200) a un première position spatiale mobile (204) ; et
o au moins un deuxième stimulus visuel (210), où au moins une partie de l'au moins un deuxième stimulus visuel (210) a une deuxième position spatiale mobile (214) ;
o dans lequel l'au moins un premier stimulus visuel (200) et l'au moins un deuxième stimulus visuel (210) sont affichés en même temps sur l'écran (102), ce qui entraîne un mouvement oculaire résultant en fonction de l'au moins un paramètre visuel ;
- au moins un dispositif de suivi oculaire (104), où l'au moins un dispositif de suivi oculaire (104) est configuré pour générer des données de suivi sur le mouvement oculaire résultant de l'au moins un œil (302) de la personne (300) ; et
- au moins un dispositif de traitement (106), où l'au moins un dispositif de traitement (106) est configuré pour déterminer au moins un paramètre visuel de l'au moins un œil (302) de la personne (300) en comparant les données de suivi, la première position spatiale mobile (204) et la deuxième position spatiale mobile (214),
dans lequel l'au moins un premier stimulus visuel (200) est un stimulus de poursuite et l'au moins un deuxième stimulus visuel (210) est un stimulus de poursuite, où la position spatiale de l'au moins un premier stimulus visuel (200) et la position spatiale de l'au moins un deuxième stimulus visuel (210) coïncident à une position spatiale coïncidente, où l'au moins un premier stimulus visuel (200) et l'au moins un deuxième stimulus visuel (210) se déplacent, à partir de la position spatiale coïncidente, de manière à ce que la position spatiale de l'au moins un premier stimulus visuel (200) et la position spatiale de l'au moins un deuxième stimulus visuel (210) ne coïncident plus,
**caractérisé en ce que**
la position spatiale coïncidente est une position spatiale correspondant, où, à cette position spatiale correspondant, au moins une partie de l'au moins un premier stimulus visuel (200) recouvre au moins une partie de l'au moins un deuxième stimulus visuel (210), ou vice versa, de telle sorte que la partie recouverte de l'au moins un deuxième stimulus visuel (210) ou la partie recouverte de l'au moins un premier stimulus visuel (200) ne soit pas perceptible par l'au moins un œil de la personne,
dans lequel l'au moins un premier stimulus visuel et l'au moins un deuxième stimulus visuel se déplacent, à partir de la position spatiale coïncidente, avec au moins une direction de mouvement spatial ou une vitesse de mouvement spatial étant différente l'une de l'autre, de sorte que l'au moins un premier stimulus visuel et l'au moins un deuxième stimulus visuel s'éloignent de la position coïncidente et/ou s'éloignent l'un de l'autre, dans lequel l'au moins un premier stimulus visuel et l'au moins un deuxième stimulus visuel diffèrent par au moins un autre paramètre de stimulus visuel choisi parmi au moins l'un des paramètres suivants : une fréquence spatiale, une gamme de fréquences spatiales ou un niveau de contraste.
